(19) Europäisches Patentamt
European Patent Office
Office européen des brevets

(11) EP 1 314 427 A1

(12) EUROPEAN PATENT APPLICATION
published in accordance with Art. 158(3) EPC

(43) Date of publication:
28.05.2003 Bulletin 2003/22

(21) Application number: 01956911.0

(22) Date of filing: 16.08.2001

(51) Int Cl.[7]: A61K 31/593, A61P 19/10, A61P 3/02

(86) International application number:
PCT/JP01/07051

(87) International publication number:
WO 02/013832 (21.02.2002 Gazette 2002/08)

(84) Designated Contracting States:
AT BE CH CY DE DK ES FI FR GB GR IE IT LI LU MC NL PT SE TR
Designated Extension States:
AL LT LV MK RO SI

(30) Priority: 17.08.2000 JP 2000247171

(71) Applicant: CHUGAI SEIYAKU KABUSHIKI KAISHA
Tokyo, 115-8543 (JP)

(72) Inventors:
- KAWASE, Akira
Gotenba-shi, Shizuoka 412-8513 (JP)
- UCHIYAMA, Yasushi
Gotenba-shi, Shizuoka 412-8513 (JP)
- TAKEDA, Satoshi
Gotenba-shi, Shizuoka 412-8513 (JP)

(74) Representative: VOSSIUS & PARTNER
Siebertstrasse 4
81675 München (DE)

# (54) REMEDIAL AGENT FOR OSTEOPOROSIS

(57) The object of the present invention is to provide a therapeutic agent for osteoporosis with a good bone mass-increasing effect, but almost no blood calcium-increasing effect.

The present invention provides a therapeutic agent for osteoporosis which comprises, as an active ingredient, a compound of Formula (1):

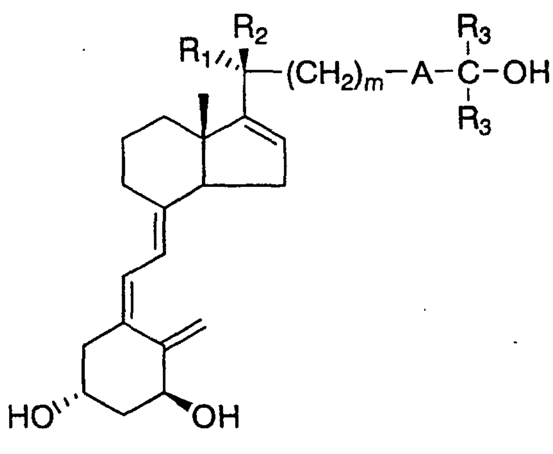

Formula (1)

wherein

$R_1$ and $R_2$ each represent a hydrogen atom or a methyl group,
$R_3$ represents a $C_1$-$C_4$ alkyl group which may be substituted with a fluorine atom,
m represents an integer of 0 to 3, and
A represents $-CH_2-CH_2-$, $-CH=CH-$ or $-C{\equiv}C-$.

EP 1 314 427 A1

## Description

### TECHNICAL FIELD

The present invention relates to therapeutic agents for osteoporosis which comprise, as an active ingredient, vitamin D derivatives having a reduced blood calcium-increasing effect, while retaining a useful bone mass-increasing effect.

### BACKGROUND ART

Active vitamin D derivatives including 1α, 25-dihydroxyvitamin $D_3$ and 1α-hydroxyvitamin $D_3$ are used as therapeutic agents for metabolic bone diseases such as osteoporosis. These active vitamin D derivatives are known to exhibit their physiological effects through vitamin D receptors. Vitamin D receptors are not only present in tissues such as small intestine, bone, kidney and parathyroid gland, but also found in various cells including immune system cells and malignant tumor cells. Thus, active vitamin D derivatives are known to have various physiological activities including: (1) regulation of calcium and bone metabolism; (2) proliferation inhibition of malignant tumor cells, epidermal cells and epithelial cells; and (3) regulation of immune system cells.

However, hypercalcemia is known as a critical side effect caused by active vitamin D derivatives in clinical use. If the blood calcium-increasing effect can be selectively reduced or eliminated from the physiological effects of active vitamin D derivatives, excellent therapeutic agents with reduced side effects can be provided for the treatment of metabolic bone diseases and novel therapeutic agents can be designed based on various physiological activities of active vitamin D derivatives.

To date, active vitamin D derivatives have been demonstrated to exhibit their blood calcium-increasing effect by the following mechanism. An active vitamin D derivative binds to the vitamin D receptor to form a complex. The complex binds to VDRE present in a promoter region of a gene for a protein involved in calcium absorption in the small intestine (e.g., Calbindin-D in charge of calcium transport in intestinal epithelial cells or Ca-ATPase mediating the transfer of the absorbed calcium to blood vessels). The binding between the complex and VDRE promotes the transcription of the gene, which in turn induces the expression of the protein. Then, the expressed protein enhances calcium absorption from the intestinal tract.

In order to provide vitamin D derivatives with an excellent bone mass-increasing effect and a reduced blood calcium-increasing effect, the inventors of the present invention focused on vitamin D derivatives having an unsaturated bond at the 16-position and a methylene group at the 22-position.

Vitamin D derivatives having an unsaturated bond at the 16-position and a methylene group at the 22-position are disclosed in, for example, W09824762, EP808833 and EP325279. However, W09824762 simply presents osteoporosis as an example of many disorders for which the vitamin D derivatives have utility; it fails to provide any specific information showing that such derivatives are effective against osteoporosis. In addition, neither EP808833 nor EP325279 teaches that such vitamin D derivatives are effective against osteoporosis.

### DISCLOSURE OF THE INVENTION

The inventors of the present invention found that a specific vitamin D derivative having an unsaturated bond at the 16-position and a methylene group at the 22-position had an excellent bone mass-increasing effect and suppressed the increase of blood Ca levels in ovariectomized (OVX) rats, a model for osteoporosis, and they finally completed the invention based on this finding.

Accordingly, the present invention provides a therapeutic agent for osteoporosis which comprises, as an active ingredient, a compound of the following Formula (1):

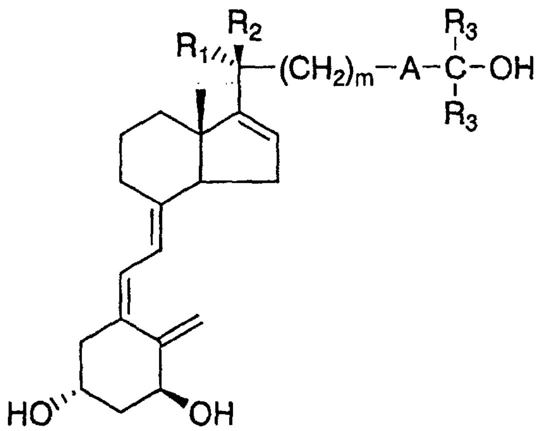

Formula (1)

wherein

$R_1$ and $R_2$ each represent a hydrogen atom or a methyl group,
$R_3$ represents a $C_1$-$C_4$ alkyl group which may be substituted with a fluorine atom,
m represents an integer of 0 to 3, and
A represents $-CH_2-CH_2-$, $-CH=CH-$ or $-C\equiv C-$.

The present invention also provides the use of a compound of Formula (1) for the preparation of a therapeutic agent for osteoporosis.

Further, the present invention provides a method for treating osteoporosis using a compound of Formula (1), for example, a method which comprises the step of administering to a patient in need of such treatment a therapeutically effective amount of a compound of Formula (1).

In addition, the present invention provides a vitamin D derivative of the following Formula (2):

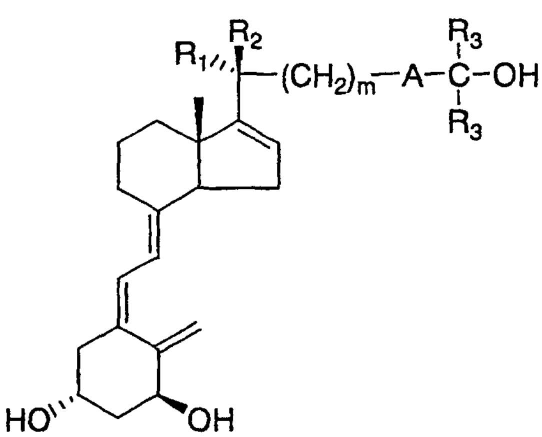

Formula (2)

wherein

$R_1$ and $R_2$ each represent a hydrogen atom or a methyl group,
$R_3$ represents a $C_1$-$C_4$ alkyl group which may be substituted with a fluorine atom,
m represents an integer of 2 or 3,
A represents $-CH=CH-$ or $-C\equiv C-$.

The present invention also provides a pharmaceutical composition comprising a compound of Formula (2) as an active ingredient.

The present invention further provides the use of a compound of Formula (2) for the preparation of a therapeutic agent for osteoporosis.

Furthermore, the present invention provides a method for treating osteoporosis using a compound of Formula (2), for example, a method which comprises the step of administering to a patient in need of such treatment a therapeutically effective amount of a compound of Formula (2).

This application claims the priority of Japanese Patent Application No. 2000-247171, the disclosure of which is hereby incorporated by reference in their entirety.

BRIEF DESCRIPTION OF DRAWINGS

Figure 1 shows the effects of the compounds according to the present invention on blood Ca level and femur bone mineral density in ovariectomized rats (**a**: blood Ca level, **b**: femur bone mineral density).

Figure 2 shows the effects of 1α,25-dihydroxyvitamin $D_3$ on urinary Ca excretion, blood Ca level and bone mineral density in ovariectomized rats (**a**: urinary Ca excretion, **b**: blood Ca level, **c**: bone mineral density).

PREFERRED MODE FOR CARRYING OUT THE INVENTION

Specific modes and implementation procedures for the present invention will be described below.

In the compound of Formula (1) or (2) according to the present invention, an alkyl group in the $C_1$-$C_4$ alkyl group which may be substituted with a fluorine atom as $R_3$ refers to a linear or branched $C_1$-$C_4$ alkyl group. Specific examples include methyl, ethyl, n-propyl, i-propyl, n-butyl, s-butyl, i-butyl and t-butyl. Preferred are methyl, ethyl, n-propyl and n-butyl, more preferred are methyl, ethyl and n-propyl, and particularly preferred are methyl and ethyl.

An alkyl group which may be substituted with a fluorine atom generally refers to a linear or branched alkyl group such as those stated above, one or more of hydrogen atoms of which may be substituted with fluorine atoms. Different types of alkyl groups may be substituted with different numbers of fluorine atoms, usually 1 to 9, preferably 3 to 7, and more preferably 3 to 5.

In a preferred mode of the compound of Formula (1) according to the present invention, $R_1$ is a hydrogen atom and $R_2$ is a methyl group.

More preferred is a compound of Formula (1) wherein $R_1$ is a hydrogen atom, $R_2$ is a methyl group, $R_3$ represents a $C_1$-$C_3$ alkyl group which may be substituted with a fluorine atom, m represents an integer of 0 to 3, and A is -CH=CH-or -C≡C-. Particularly preferred is a compound of Formula (1) wherein $R_1$ is a hydrogen atom, $R_2$ is a methyl group, $R_3$ represents a $C_1$-$C_3$ alkyl group, m represents an integer of 2 or 3, and A is -CH=CH- or -C≡C-. Even more preferred is 1α,3β-dihydroxy-20(S)-(5-ethyl-5-hydroxy-3-heptynyl)-9,10-secopregna-5,7,10(19),16-tetraene.

Also preferred is a compound of Formula (1) wherein $R_1$ is a hydrogen atom, $R_2$ is a methyl group, $R_3$ represents a $C_1$-$C_3$ alkyl group which may be substituted with a fluorine atom, m represents an integer of 0 to 3, and A is $-CH_2$-$CH_2$-. Particularly preferred is a compound of Formula (1) wherein $R_1$ is a hydrogen atom, $R_2$ is a methyl group, $R_3$ represents a $C_1$-$C_3$ alkyl group, m represents 0, and A is $-CH_2$-$CH_2$-. Even more preferred is 1α, 3β-dihydroxy-20 (S)-(3-ethyl-3-hydroxypentyl)-9,10-secopregna-5,7,10(19),16-tetraene.

In a preferred mode of the vitamin D derivative of Formula (2) according to the present invention, $R_1$ is a hydrogen atom and $R_2$ is a methyl group. Particularly preferred is 1α,3β-dihydroxy-20(S)-(5-ethyl-5-hydroxy-3-heptynyl)-9,10-secopregna-5,7,10(19),16-tetraene.

The following Tables 1 to 3 present specific, but non-limiting examples of the compound of Formula (1) or (2) according to the present invention.

Table 1

| $R_1$ | $R_2$ | m | A | $R_3$ |
|---|---|---|---|---|
| $CH_3$ | H | 0 | $-CH_2$-$CH_2$- | $CH_3$ |
| $CH_3$ | H | 0 | $-CH_2$-$CH_2$- | $CH_2CH_3$ |
| $CH_3$ | H | 1 | $-CH_2$-$CH_2$- | $CH_3$ |
| $CH_3$ | H | 1 | $-CH_2$-$CH_2$- | $CH_2CH_3$ |
| $CH_3$ | H | 1 | -CH=CH-(E) | $CH_3$ |
| $CH_3$ | H | 1 | -CH=CH-(E) | $CH_2CH_3$ |
| $CH_3$ | H | 1 | -CH=CH-(Z) | $CH_3$ |
| $CH_3$ | H | 1 | -CH=CH-(Z) | $CH_2CH_3$ |
| $CH_3$ | H | 1 | -C≡C- | $CH_3$ |
| $CH_3$ | H | 1 | -C≡C- | $CH_2CH_3$ |

Table 1 (continued)

| $R_1$ | $R_2$ | m | A | $R_3$ |
|---|---|---|---|---|
| $CH_3$ | H | 2 | $-CH_2-CH_2-$ | $CH_3$ |
| $CH_3$ | H | 2 | $-CH_2-CH_2-$ | $CH_2CH_3$ |
| $CH_3$ | H | 2 | -CH=CH-(E) | $CH_3$ |
| $CH_3$ | H | 2 | -CH=CH-(E) | $CH_2CH_3$ |
| $CH_3$ | H | 2 | -CH=CH-(Z) | $CH_3$ |
| $CH_3$ | H | 2 | -CH=CH-(Z) | $CH_2CH_3$ |
| $CH_3$ | H | 2 | -C≡C- | $CH_3$ |
| $CH_3$ | H | 2 | -C≡C- | $CH_2CH_3$ |
| $CH_3$ | H | 3 | $-CH_2-CH_2-$ | $CH_3$ |
| $CH_3$ | H | 3 | -CH2-CH2- | $CH_2CH_3$ |
| $CH_3$ | H | 3 | -CH=CH-(E) | $CH_3$ |
| $CH_3$ | H | 3 | -CH=CH-(E) | $CH_2CH_3$ |
| $CH_3$ | H | 3 | -CH=CH-(Z) | $CH_3$ |
| $CH_3$ | H | 3 | -CH=CH-(Z) | $CH_2CH_3$ |
| $CH_3$ | H | 3 | -C≡C- | $CH_3$ |
| $CH_3$ | H | 3 | -C≡C- | $CH_2CH_3$ |
| H | $CH_3$ | 0 | $-CH_2-CH_2-$ | $CH_3$ |
| H | $CH_3$ | 0 | $-CH_2-CH_2-$ | $CF_3$ |
| H | $CH_3$ | 0 | $-CH_2-CH_2-$ | $CH_2CH_3$ |
| H | $CH_3$ | 0 | $-CH_2-CH_2-$ | $CH_2CF_3$ |

Table 2

| $R_1$ | $R_2$ | m | A | $R_3$ |
|---|---|---|---|---|
| H | $CH_3$ | 0 | $-CH_2-CH_2-$ | $CF_2CF_3$ |
| H | $CH_3$ | 1 | $-CH_2-CH_2-$ | $CH_3$ |
| H | $CH_3$ | 1 | $-CH_2-CH_2-$ | $CH_2CH_3$ |
| H | $CH_3$ | 1 | $-CH_2-CH_2-$ | $CH_2CH_2CH_3$ |
| H | $CH_3$ | 1 | -CH=CH-(E) | $CH_3$ |
| H | $CH_3$ | 1 | -CH=CH-(E) | $CH_2CH_3$ |
| H | $CH_3$ | 1 | -CH=CH-(E) | $CH_2CH_2CH_3$ |
| H | $CH_3$ | 1 | -CH=CH-(Z) | $CH_3$ |
| H | $CH_3$ | 1 | -CH=CH-(Z) | $CH_2CH_3$ |
| H | $CH_3$ | 1 | -CH=CH-(Z) | $CH_2CH_2CH_3$ |
| H | $CH_3$ | 1 | -C≡C- | $CH_3$ |
| H | $CH_3$ | 1 | -C≡C- | $CH_2CH_3$ |
| H | $CH_3$ | 1 | -C≡C- | $CH_2CH_2CH_3$ |
| H | $CH_3$ | 2 | $-CH_2-CH_2-$ | $CH_3$ |
| H | $CH_3$ | 2 | $-CH_2-CH_2-$ | $CH_2CH_3$ |
| H | $CH_3$ | 2 | $-CH_2-CH_2-$ | $CH_2CH_2CH_3$ |
| H | $CH_3$ | 2 | -CH=CH-(E) | $CH_3$ |
| H | $CH_3$ | 2 | -CH=CH-(E) | $CF_3$ |
| H | $CH_3$ | 2 | -CH=CH-(E) | $CH_2CH_3$ |
| H | $CH_3$ | 2 | -CH=CH-(E) | $CH_2CF_3$ |
| H | $CH_3$ | 2 | -CH=CH-(E) | $CF_2CF_3$ |
| H | $CH_3$ | 2 | -CH=CH-(E) | $CH_2CH_2CH_3$ |
| H | $CH_3$ | 2 | -CH=CH-(E) | $CH_2CH_2CH_2CH_3$ |
| H | $CH_3$ | 2 | -CH=CH-(Z) | $CH_3$ |
| H | $CH_3$ | 2 | -CH=CH-(Z) | $CF_3$ |

Table 2 (continued)

| $R_1$ | $R_2$ | m | A | $R_3$ |
|---|---|---|---|---|
| H | $CH_3$ | 2 | -CH=CH-(Z) | $CH_2CH_3$ |
| H | $CH_3$ | 2 | -CH=CH-(Z) | $CH_2CF_3$ |
| H | $CH_3$ | 2 | -CH=CH-(Z) | $CF_2CF_3$ |
| H | $CH_3$ | 2 | -CH=CH-(Z) | $CH_2CH_2CH_3$ |
| H | $CH_3$ | 2 | -CH=CH-(Z) | $CH_2CH_2CH_2CH_3$ |

Table 3

| $R_1$ | $R_2$ | m | A | $R_3$ |
|---|---|---|---|---|
| H | $CH_3$ | 2 | -C≡C- | $CH_3$ |
| H | $CH_3$ | 2 | -C≡C- | $CF_3$ |
| H | $CH_3$ | 2 | -C≡C- | $CH_2CH_3$ |
| H | $CH_3$ | 2 | -C≡C- | $CH_2CF_3$ |
| H | $CH_3$ | 2 | -C≡C- | $CF_2CF_3$ |
| H | $CH_3$ | 2 | -C≡C- | $CH_2CH_2CH_3$ |
| H | $CH_3$ | 2 | -C≡C- | $CH_2CH_2CH_2$ $CH_3$ |
| H | $CH_3$ | 3 | -CH2-CH2- | $CH_3$ |
| H | $CH_3$ | 3 | $-CH_2-CH_2-$ | $CH_2CH_3$ |
| H | $CH_3$ | 3 | $-CH_2-CH_2-$ | $CH_2CH_2CH_3$ |
| H | $CH_3$ | 3 | -CH=CH-(E) | $CH_3$ |
| H | $CH_3$ | 3 | -CH=CH-(E) | $CH_2CH_3$ |
| H | $CH_3$ | 3 | -CH=CH-(E) | $CH_2CH_2CH_3$ |
| H | $CH_3$ | 3 | -CH=CH-(Z) | $CH_3$ |
| H | $CH_3$ | 3 | -CH=CH-(Z) | $CH_2CH_3$ |
| H | $CH_3$ | 3 | -CH=CH-(Z) | $CH_2CH_2CH_3$ |
| H | $CH_3$ | 3 | -C≡C- | $CH_3$ |
| H | $CH_3$ | 3 | -C≡C- | $CH_2CH_3$ |
| H | $CH_3$ | 3 | -C≡C- | $CH_2CH_2CH_3$ |

The compounds of the present invention may be prepared, for example, in the following ways.

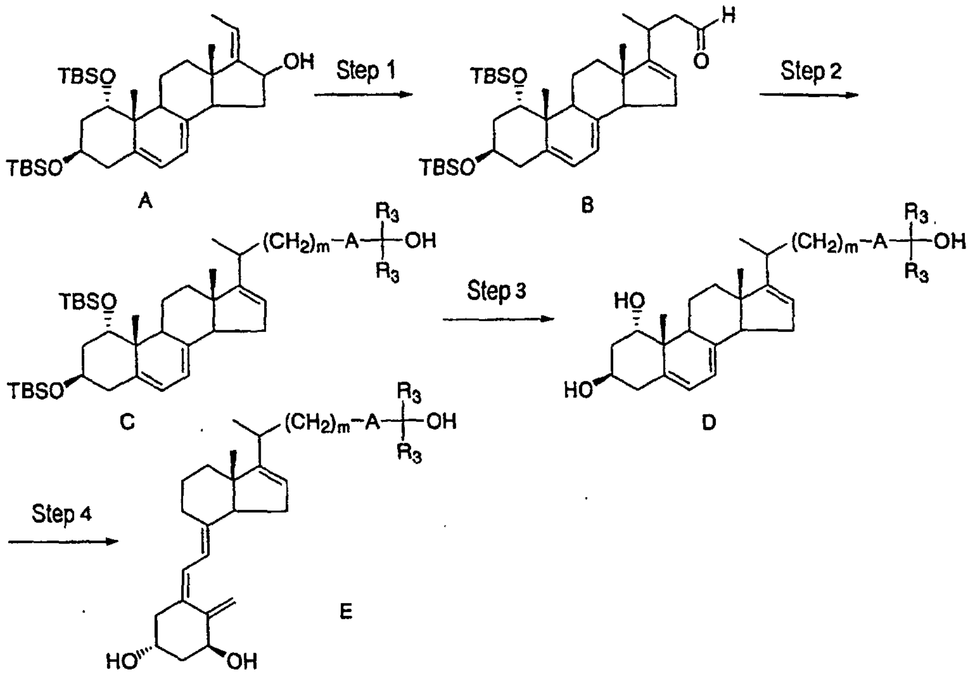


A B C D E

In the above reaction scheme, $R_3$ represents a $C_1$-$C_4$ alkyl group which may be substituted with a fluorine atom, m represents an integer of 0 to 3, and A represents $-CH_2-CH_2-$, -CH=CH- or -C≡C-, provided that m represents an integer of 2 or 3 and A represents -CH=CH- or -C≡C- if the final compound E has Formula (2).

<u>Step 1 (Synthesis and thermal rearrangement of vinyl ether)</u>

In an alkyl vinyl ether, Compound A described in JP 10-231284 A is treated with an acid catalyst to give a vinyl ether thereof. An alkyl vinyl ether usually available for use is ethyl vinyl ether. Examples of an acid catalyst include alkylsulfonic acids and divalent mercury salts, such as methanesulfonic acid, paratoluenesulfonic acid, camphorsulfonic acid, boron trifluoride-diethyl ether complex, mercury(II) chloride and mercury(II) acetate, with mercury(II) acetate being preferred. The reaction may be performed at any temperature as long as the reaction can proceed, usually at -20°C to 100°C, preferably at room temperature.

The vinyl ether thus prepared is subjected to thermal rearrangement in an appropriate solvent to give Compound B. Any solvent may be used for this reaction. Examples include hydrocarbon solvents, ether solvents, halogenated solvents, ketone/ester/amide solvents, sulfoxide solvents and nitrile solvents, such as hexane, benzene, toluene, diethyl ether, tetrahydrofuran, 1,4-dioxane, 1,2-dimethoxyethane, dichloromethane, 1,2-dichloroethane, chloroform, carbon tetrachloride, acetone, ethyl acetate, N,N-dimethylformamide, N,N-dimethylacetamide, 1,3-dimethyl-2-imidazolidinone. 1,3-dimethyl-3,4,5,6-tetrahydro-2(1H)-pyrimidinone, dimethyl sulfoxide and acetonitrile. Preferred are benzene, toluene, 1,4-dioxane, N,N-dimethylformamide, 1,3-dimethyl-2-imidazolidinone and dimethyl sulfoxide, and more preferred are toluene and 1,3-dimethyl-2-imidazolidinone. The reaction may be performed at any temperature as long as the reaction can proceed, usually at a temperature ranging from room temperature to 200°C, preferably at 80°C to 140°C.

This step allows the specific synthesis of the configuration at the 20-position depending on the configuration of the 16-position hydroxy group. Namely, compounds having 20R- and 20S-configurations can be specifically synthesized from 16α- and 16β-hydroxy forms, respectively.

<u>Step 2 (Introduction of a side chain)</u>

The introduction of a side chain may be accomplished as shown in the following Introduction Examples 1 to 3.

(Introduction Example 1)

Example 1

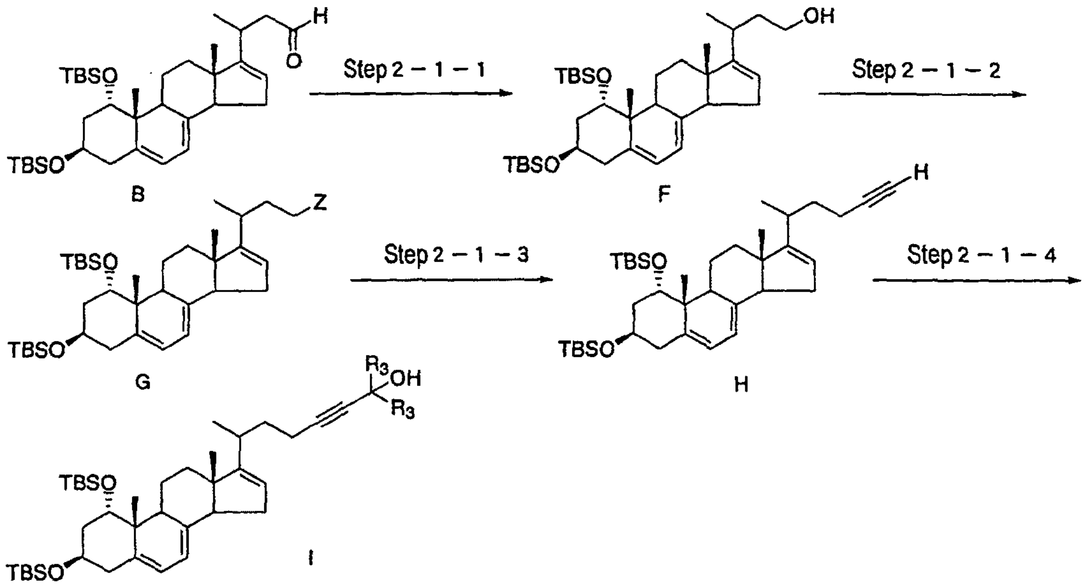


In the above reaction scheme, $R_3$ represents a $C_1$-$C_4$ alkyl group which may be substituted with a fluorine atom and Z represents a leaving group.

Step 2-1-1 (Reduction)

Compound B can be reduced to give Compound F according to standard procedures (Burke, S. D. and Danheiser, R. L., Handbook of Reagent for Organic Synthesis: Oxidizing and Reducing Agents, John Wiley and Sons, Chichester, UK, 1999). Preferably, sodium borohydride is used as a reducing agent.

Step 2-1-2 (Conversion into a leaving group)

In the reaction scheme, Z represents a leaving group, for example, a chlorine atom, a bromine atom, an iodine atom, a mesyloxy group and a tosyloxy group, with a mesyloxy group and a tosyloxy group being preferred. Compound F can be converted into Compound G according to standard procedures (Larock, R. C. Comprehensive Organic Transformations, 2nd Ed.; Wiley-VCH; New York, 1999).

Step 2-1-3 (Introduction of an acetylene unit)

In the presence of an appropriate solvent, Compound G is treated with a metal acetylide to give Compound H. Examples of a metal acetylide available for use include lithium acetylide, lithium acetylide-ethylenediamine complex, sodium acetylide, potassium acetylide and alkylmagnesium acetylide, with lithium acetylide and lithium acetylide-ethylenediamine complex being preferred. Examples of a solvent available for use include ether solvents, sulfoxide solvents and amide solvents, such as diethyl ether, tetrahydrofuran, 1,4-dioxane, 1,2-dimethoxyethane, N,N-dimethylformamide, N,N-dimethylacetamide, 1,3-dimethyl-2-imidazolidinone, 1,3-dimethyl-3,4,5,6-tetrahydro-2(1H)-pyrimidinone and dimethyl sulfoxide, with tetrahydrofuran and dimethyl sulfoxide being preferred. These solvents may also be used in combination. The reaction may be performed at any temperature as long as the reaction can proceed, usually at a temperature ranging from -20°C to 150°C, preferably from room temperature to 60°C.

Step 2-1-4 (Alkylation)

In an appropriate solvent, Compound H is treated with a base followed by $(R_3)_2CO$ to give Compound I.

Examples of a base include metal alkyls, alkali metal hydrides, alkali metal alkoxides and metal dialkylamides; n-butyllithium, s-butyllithium. t-butyllithium, methyllithium, phenyllithium, methylmagnesium bromide, methylmagnesium chloride, methylmagnesium iodide, ethylmagnesium bromide, ethylmagnesium iodide, isopropylmagnesium bromide, sodium hydride, potassium hydride, sodium methoxide, potassium t-butoxide, lithium amide, lithium diethylamide, lithium diisopropylamide and lithium bis(trimethylsilyl)amide are preferred, with n-butyllithium and ethylmagnesium iodide being more preferred. The reaction is performed in an ether solvent, preferably in diethyl ether, tetrahydrofuran, 1,4-dioxane or 1,2-dimethoxyethane, more preferably in tetrahydrofuran. The reaction may be performed at any temperature as long as the reaction can proceed, usually at a temperature ranging from -78°C to 60°C, preferably from -20°C to room temperature.

(Introduction Example 2)

Example 2

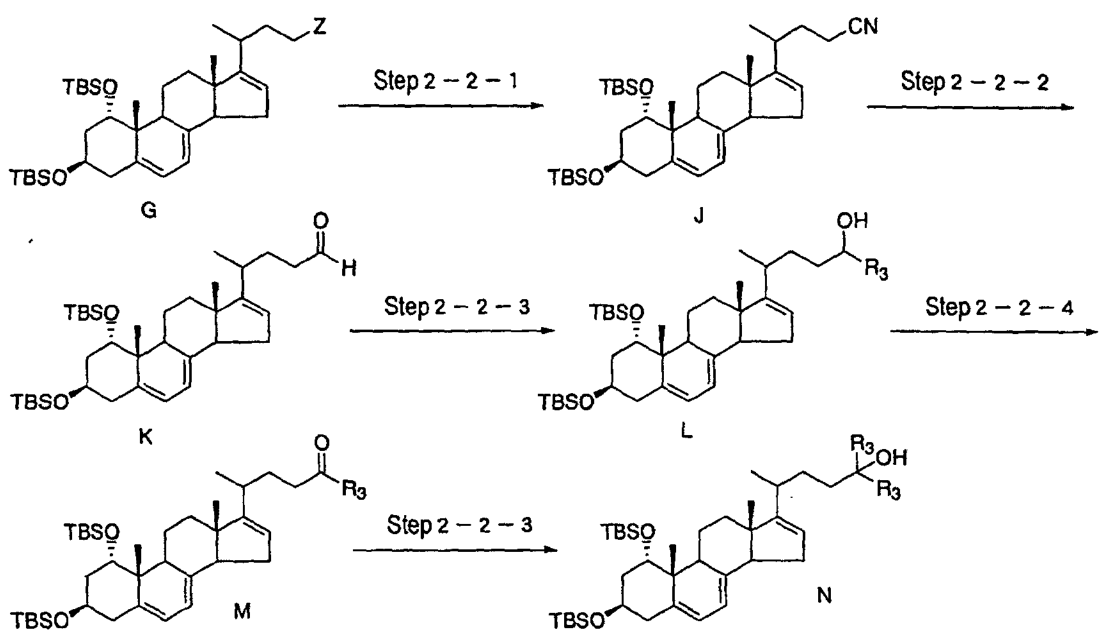


In the above reaction scheme, $R_3$ represents a $C_1$-$C_4$ alkyl group which may be substituted with a fluorine atom and Z represents a leaving group.

Step 2-2-1 (Addition of one carbon atom)

In an appropriate solvent, Compound G from Step 2-1-2 of Introduction Example 1 is treated with a metallocyanide compound to give Compound J. A metallocyanide compound usually available for use is sodium cyanide or potassium cyanide. Examples of a solvent usually available for use include ether solvents, amide solvents and sulfoxide solvents, such as diethyl ether, tetrahydrofuran, 1,4-dioxane. 1,2-dimethoxyethane, N,N-dimethylformamide, N.N-dimethylacetamide, 1,3-dimethyl-2-imidazolidinone, 1,3-dimethyl-3,4,5,6-tetrahydro-2(1H)-pyrimidinone and dimethyl sulfoxide. Preferred are N,N-dimethylformamide and dimethyl sulfoxide, and more preferred is N,N-dimethylformamide. The reaction may be performed at any temperature as long as the reaction can proceed, preferably at a temperature ranging from 0°C to 150°C, more preferably from room temperature to 100°C.

Step 2-2-2 (Reduction)

Compound J can be converted into Compound K according to standard procedures (Burke, S. D. and Danheiser, R. L., Handbook of Reagent for Organic Synthesis: Oxidizing and Reducing Agents, John Wiley and Sons, Chichester, UK, 1999). Preferably, diisobutylaluminum hydride is used as a reducing agent.

Step 2-2-3 (Alkylation)

In an appropriate solvent, Compound K is treated with an alkylating agent corresponding to the side chain $R_3$:

$$R_3\text{-M}$$

(wherein M represents lithium or a magnesium halide, and $R_3$ represents a $C_1$-$C_4$ alkyl group which may be substituted with a fluorine atom) to give Compound L. Preferably, a magnesium halide is used as a metal. Examples of a solvent usually available for use include ether solvents, such as diethyl ether, tetrahydrofuran, 1,4-dioxane and 1,2-dimethoxyethane, with diethyl ether and tetrahydrofuran being preferred. The reaction is usually performed at a temperature ranging from -20°C to 50°C, preferably from 0°C to room temperature.

Step 2-2-4 (Oxidation)

Compound L can be oxidized to give Compound M according to standard procedures (Burke, S. D. and Danheiser, R. L., Handbook of Reagent for Organic Synthesis: Oxidizing and Reducing Agents, John Wiley and Sons, Chichester, UK, 1999). Examples of a preferred oxidizing agent include dimethyl sulfoxide/oxalyl chloride, dimethyl sulfoxide/triphosgene, dimethyl sulfoxide/sulfur trioxide-pyridine complex, pyridinium dichromate, pyridinium chlorochromate and tetra-n-propylammonium perruthenate/4-methylmorpholine N-oxide.

Compound M thus prepared is further treated in the same manner as Step 2-2-3 to give Compound N.

(Introduction Example 3)

Example 3

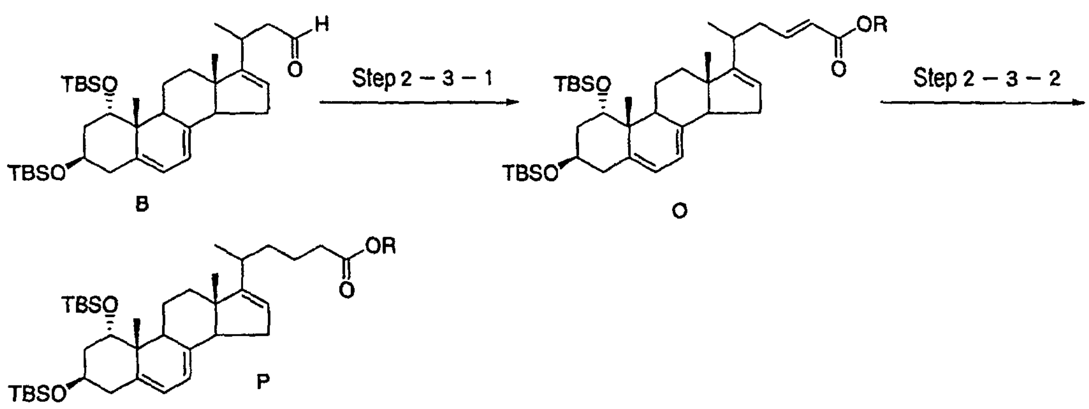

B
O
P

In the above reaction scheme, R represents a lower alkyl group.

Step 2-3-1 (Addition of two carbon atoms)

In an appropriate solvent, Compound B is treated with a reagent:

$$(RO)_3P(O)CH_2CO_2R'$$

(wherein R and R' each represent a lower alkyl group) in the presence of a base to give Compound O. Examples of a base include tertiary amines, metal alkyls, alkali metal hydrides, alkali metal alkoxides and metal dialkylamide, such as triethylamine, diisopropylethylamine, pyridine, n-butyllithium, s-butyllithium, t-butyllithium, methyllithium, phenyllithium, methylmagnesium bromide, methylmagnesium chloride, methylmagnesium iodide, ethylmagnesium bromide, ethylmagnesium iodide, isopropylmagnesium bromide, sodium hydride, potassium hydride, sodium methoxide, potassium t-butoxide, lithium amide, lithium diethylamide, lithium diisopropylamide and lithium bis(trimethylsilyl)amide. Preferred are triethylamine, diisopropylethylamine, n-butyllithium and sodium hydride, and more preferred is triethylamine. The treatment may be performed in the presence of an alkali metal chloride together with a base. Examples of an alkali metal chloride include lithium chloride, lithium bromide, lithium iodide, sodium chloride, sodium bromide, sodium iodide, potassium chloride, potassium bromide and potassium iodide, with lithium chloride and lithium bromide being preferred, lithium bromide being more preferred. Examples of a solvent usually available for use include ether solvents, such as diethyl ether, tetrahydrofuran, 1,4-dioxane and 1,2-dimethoxyethane, with tetrahydrofuran being preferred. The reaction is usually performed at a temperature ranging from - 20°C to 50°C, preferably from 0°C to room temperature.

Step 2-3-2 (Selective reduction of an unsaturated bond)

In an ether or alcohol solvent, Compound O is reduced with sodium borohydride in the presence of a catalytic amount of a nickel(II) or cobalt(II) salt to give Compound P. Examples of a nickel(II) or cobalt(II) salt include nickel(II) acetate tetrahydrate, nickel(II) bromide trihydrate, nickel(II) chloride hexahydrate, nickel(II) iodide hexahydrate, cobalt (II) acetate tetrahydrate, cobalt(II) bromide hexahydrate and cobalt(II) chloride hexahydrate, with nickel(II) chloride hexahydrate and cobalt(II) chloride hexahydrate being preferred. Examples of a solvent available for use include ether solvents and alcohol solvents, such as diethyl ether, tetrahydrofuran, 1,4-dioxane, 1,2-dimethoxyethane, methanol, ethanol, n-propanol and i-propanol, with tetrahydrofuran, 1,2-dimethoxyethane, methanol and ethanol being preferred. Further, these solvents may be used in combination. The reaction is usually performed at a temperature ranging from - 20°C to 50°C, preferably from 0°C to room temperature. This step allows the selective reduction of the unsaturated bond present at the side chain among four unsaturated bonds in Compound O.

As shown in the following reaction schemes, Compounds O and P thus prepared can then be subjected to Step 2-2-3 mentioned above to give Compounds Q and R, respectively.

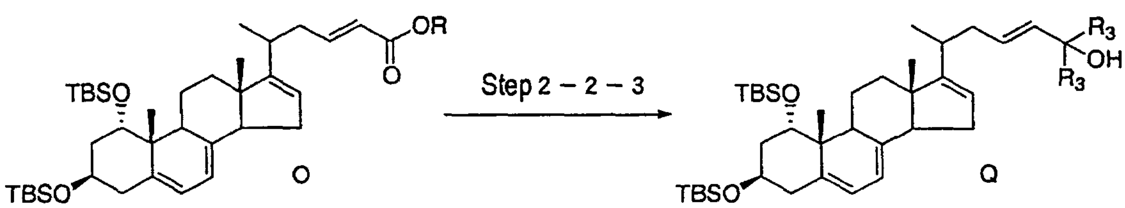

O
Q

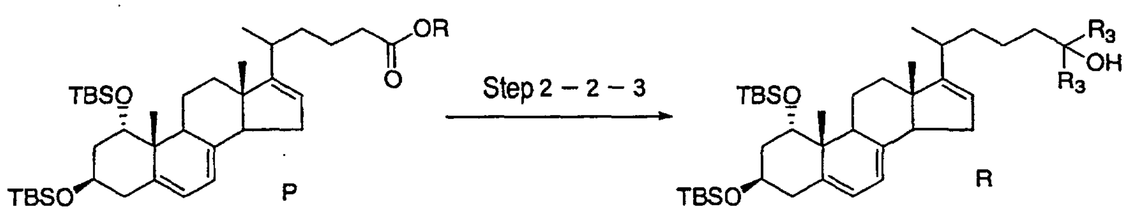

P
R

In the above reaction schemes, $R_3$ represents a $C_1$-$C_4$ alkyl group which may be substituted with a fluorine atom and R represents a lower alkyl group.

Step 3 (Deprotection)

According to standard procedures, protecting groups are removed from Compound C prepared in the above.

Examples of a deprotection reagent available for use include hydrochloric acid, sulfuric acid, acetic acid, acidic ion exchange resins, tetra-n-butylammonium fluoride, hydrogen fluoride/pyridine, hydrogen fluoride/triethylamine and hydrofluoric acid, with acidic ion exchange resins and tetra-n-butylammonium fluoride being preferred. The deprotection is usually performed in an ether solvent, preferably in tetrahydrofuran. The reaction temperature for deprotection will vary according to the type of substrate, but it is usually in the range of room temperature to 65°C.

Step 4 (Photoreaction and thermal isomerization)

According to standard procedures, Compound D is subjected to photoreaction and thermal isomerization to give Compound E. Steps 2, 3 and 4 may be performed in any order, provided that Step 3 does not precede Step 2.
In addition, the above Steps 1 to 3 may also start with the compound as shown below (which can be found in JP 10-231284 A) instead of Compound A.

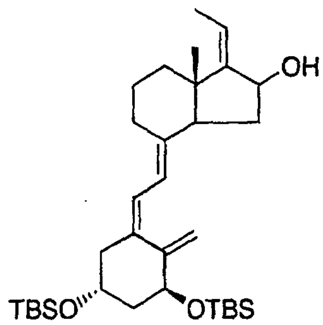

In the individual synthesis steps mentioned above, the respective intermediates and final products can be purified by standard techniques such as silica gel column chromatography, thin layer chromatography and recrystallization.
As shown in the Examples below, the compound of Formula (1) or (2) thus prepared is useful as a therapeutic agent for osteoporosis having a reduced blood calcium-increasing effect, while retaining a bone mass-increasing effect attributed to vitamin D.
A therapeutic agent for osteoporosis comprising the compound of the present invention may be administered by oral, subcutaneous, intramuscular, intravenous or percutaneous route, preferably by oral route.
The therapeutic agent for osteoporosis comprising the compound of the present invention may be formulated into the desired dosage form such as tablets, granules, fine granules, capsules, powders, injections, solutions, suspensions, emulsions, transdermal absorption systems or suppositories in combination with known pharmaceutically acceptable carriers, excipients, disintegrating agents, lubricants, binders, flavoring agents, coloring agents and the like.
The dose of the therapeutic agent for osteoporosis comprising the compound of the present invention as an active ingredient can be determined as appropriate for the condition, physique, diathesis, age and sex of a patient, the intended route of administration, or the type of dosage form, etc. For oral administration, the agent is generally used in an amount as the active ingredient of 0.01 µg/day to 10,000 µg/day, preferably 0.1 µg/day to 1,000 µg/day, in one to three divided doses per day.

EXAMPLES

The present invention will be further described in the following Examples, which are provided for illustrative purposes only and are not intended to limit the scope of the invention.

(Example 1) Preparation of 20(S)-1α,3β-bis(t-butyldimethylsilyloxy)-24-norchola-5,7,16-trien-23-al

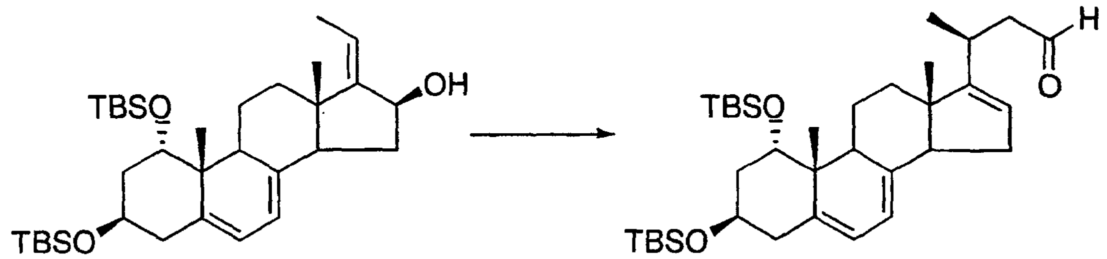

1α,3β-Bis(t-butyldimethylsilyloxy)-16β-hydroxypregna-5,7,17(E)-triene (1.50 g, 2.68 mmol) was dissolved in ethyl vinyl ether (35 ml), followed by addition of mercury(II) acetate (682 mg, 2.14 mmol) and stirring at room temperature for 38 hours. The reaction mixture was poured into 5% aqueous potassium hydroxide and extracted with hexane. The organic layer was washed with saturated brine, dried over anhydrous magnesium sulfate, and then evaporated under reduced pressure to remove the solvent. The resulting residue was dissolved in 1,3-dimethyl-2-imidazolidinone (50 ml) and stirred at 110°C for 4 hours. After cooling to room temperature, the reaction mixture was poured into water and extracted twice with ethyl acetate. The combined organic layers were washed with water and saturated brine, dried over anhydrous magnesium sulfate, and then evaporated under reduced pressure to remove the solvent. The resulting residue was purified by silica gel column chromatography (hexane:ethyl acetate = 30:1) to give the titled compound (1.08 g, 69%) as a white solid.

$^1$H NMR δ: 0.05(s, 3H), 0.07(s, 6H), 0.11(s, 3H), 0.84(8, 3H), 0.88(s, 18H), 0.94(s, 3H), 1.13(d, J=6.9Hz, 3H), 2.56-2.67(m, 1H), 2.73-2.91(m, 1H). 3.69-3.74(m, 1H), 3.98-4.13(m, 1H), 5.37-5.44(m, 2H), 5.59-5.63(m, 1H). 9.72(t, J=2.1Hz, 1H).

(Example 2) Preparation of 20(S)-1α,3β-bis(t-butyldimethylsilyloxy)-23-(p-toluenesulfonyloxy)-24-norchola-5,7,16-triene

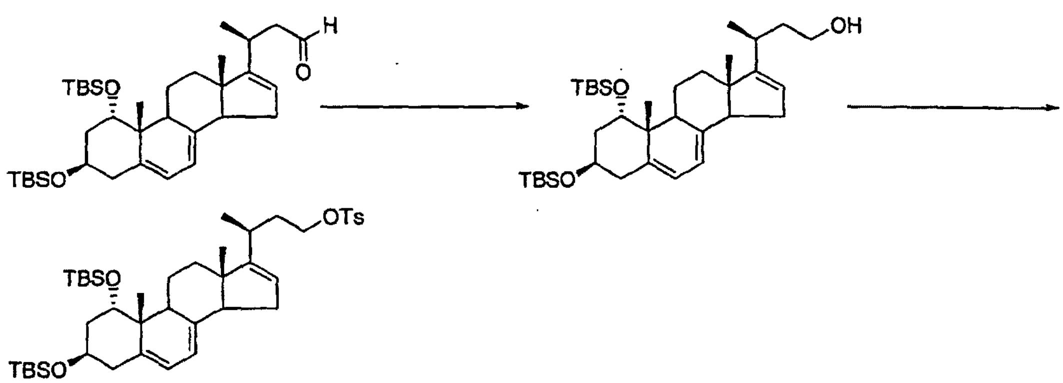

20(S)-1α,3β-Bis(t-butyldimethylsilyloxy)-24-norchola-5,7,16-trien-23-al (763 mg, 1.30 mmol) was dissolved in a mixed solvent of methanol (3 ml) and tetrahydrofuran (7 ml), and sodium borohydride (98.4 mg, 2.60 mmol) was slowly added thereto at 0°C. After stirring at room temperature for 1 hour, the reaction mixture was poured into water and extracted with ethyl acetate. The extracted solution was washed with saturated brine, dried over magnesium sulfate, and then evaporated under reduced pressure to remove the solvent. The resulting residue was dissolved in dichloromethane (5 ml) and cooled to 0°C after addition of pyridine (0.51 ml, 6.35 mmol). At this temperature, p-toluenesulfonyl chloride (364 mg, 1.91 mmol) was further added and stirred at the same temperature for 12 hours. The reaction mixture was diluted with ethyl acetate, washed sequentially with ice-cold 0.5 M hydrochloric acid, saturated aqueous

sodium bicarbonate and saturated brine, and then dried over magnesium sulfate. After evaporation under reduced pressure to remove the solvent, the resulting residue was purified by silica gel column chromatography (hexane:dichloromethane = 9:1 and then 3:2) to give the titled compound (829 mg, 88%) as a white foam.

$^1$H NMR δ: 0.06(s, 3H), 0.07(s, 3H), 0.09(s, 3H), 0.12(s, 3H), 0.77(s, 3H), 0.89(s, 9H), 0.90(s, 9H), 0.94(s, 3H), 1.00(d, J=6.9Hz, 3H), 2.45(s, 3H), 2.78-2.90(m, 1H), 3.67-3.75(m, 1H), 3.98-4.13(m, 3H), 5.26-5.31(m, 1H), 5.34-5.41(m, 1H), 5.56-5.63(m, 1H), 7.34(d, J=8.2Hz, 2H), 7.78(d, J=8.2Hz, 2H).

(Example 3) Preparation of 1α,3β-bis(t-butyldimethylsilyloxy)-20(S)-(3-butynyl)pregna-5,7,16-triene

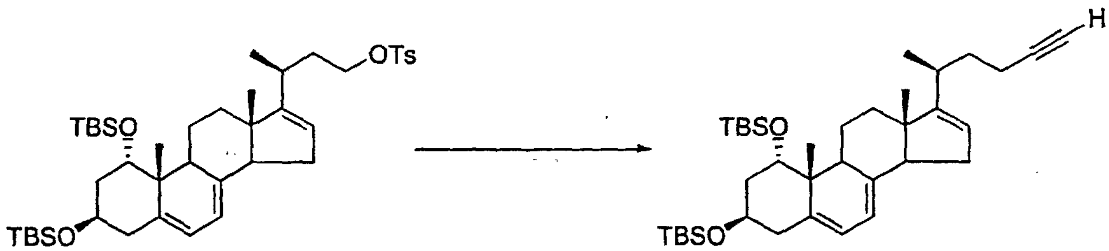

20(S)-1α,3β-Bis(t-butyldimethylsilyloxy)-23-(p-toluenesulfonyloxy)-24-norchola-5,7,16-triene (152 mg, 0.205 mmol) was dissolved in a mixed solvent of tetrahydrofuran (0.5 ml) and dimethyl sulfoxide (1 ml), followed by addition of lithium acetylide-ethylenediamine complex (62.9 mg, 0.615 mmol) and stirring at room temperature for 3 hours. The reaction mixture was poured into ice-cold water and extracted with hexane. The organic layer was washed with saturated brine and dried over magnesium sulfate. After evaporation under reduced pressure to remove the solvent, the resulting residue was purified by silica gel column chromatography (hexane:dichloromethane = 5:1) to give the titled compound (96.9 mg, 79%) as a colorless oil.

$^1$H NMR δ: 0.06(s, 3H), 0.07(s, 3H), 0.08(s, 3H), 0.11(s, 3H), 0.81(s, 3H), 0.89(s, 18H), 0.94(s, 3H), 1.07(d, J=6.9Hz, 3H), 2.78-2.92(m, 1H), 3.66-3.77(m, 1H), 3.95-4.15(m, 1H), 5.32-5.47(m, 2H), 5.56-5.65(m, 1H).

(Example 4) Preparation of 1α3β-bis(t-butyldimethylsilyloxy)-20(S)-(5-ethyl-5-hydroxy-3-heptynyl)pregna-5,7,16-triene

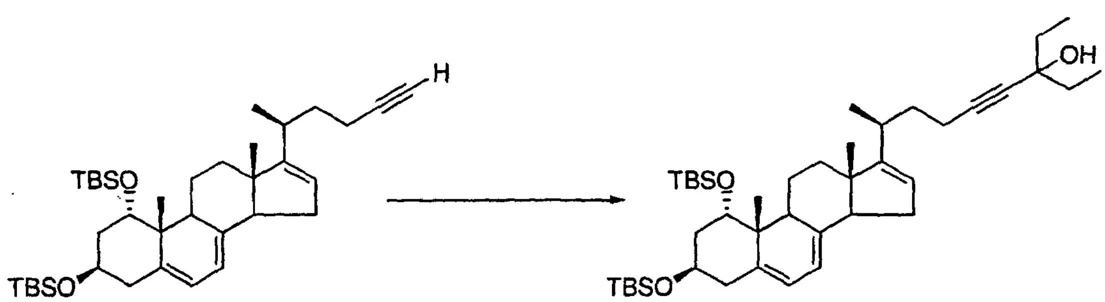

To a solution of 1α,3β-bis(t-butyldimethylsilyloxy)-20(S)-(3-butynyl)pregna-5,7,16-triene (96.9 mg, 0.162 mmol) in tetrahydrofuran (2 ml), 1.47 M n-butyllithium (221 μl, 0.325 mmol) was added dropwise at 0°C and stirred at the same temperature for 15 minutes. To this solution, 3-pentanone (49.1 μl, 0.486 mmol) was added and stirred at 0°C for 2 hours. Ice-cold water was added dropwise to the reaction mixture, which was then extracted with ethyl acetate. The extracted solution was washed with saturated brine, dried over anhydrous magnesium sulfate, and then evaporated under reduced pressure to remove the solvent. The resulting residue was purified by silica gel column chromatography (hexane:ethyl acetate = 20: 1) to give the titled compound (50.1 mg, 45%) as a colorless oil.

$^1$H NMR δ: 0.06(s, 3H), 0.07(s, 3H), 0.07(s, 3H), 0.11(S, 3H), 0.81(s, 3H), 0.89(s, 18H), 0.95(s, 3H), 2.80-2.93(m, 1H), 3.68-3.78(m, 1H), 3.98-4.15(m, 1H), 5.35-5.47(m, 2H), 5.57-5.66(m, 1H).

(Example 5) Preparation of 1α,3β-dihydroxy-20(S)-(5-ethyl-5-hydroxy-3-heptynyl)pregna-5,7,16-triene

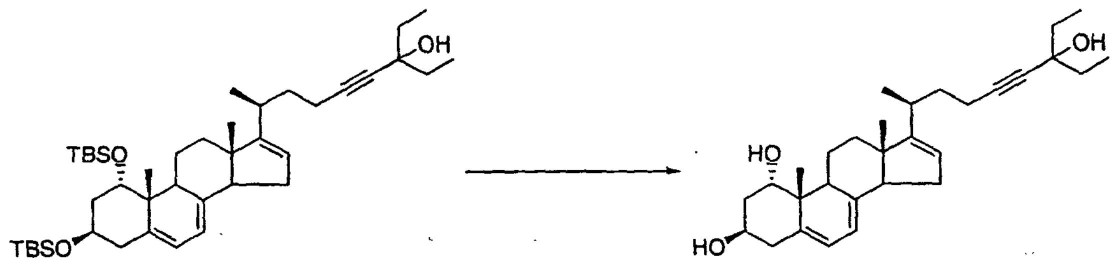

To a solution of 1α,3β-bis(t-butyldimethylsilyloxy)-20(S)-(5-ethyl-5-hydroxy-3-heptynyl)pregna-5,7,16-triene (62.5 mg, 0.091 mmol) in tetrahydrofuran (1 ml), a 1M tetrahydrofuran solution of tetra-n-butylammonium fluoride (915 μl, 0.915 mmol) was added, followed by heating at reflux for 6 hours. After cooling to room temperature, the reaction mixture was diluted with ethyl acetate, washed with water and saturated brine, and then dried over anhydrous magnesium sulfate. After evaporation under reduced pressure to remove the solvent, the resulting residue was purified by silica gel column chromatography (hexane:ethyl acetate = 1:1 and then 1:3) to give the titled compound (38.0 mg, 92%) as a colorless oil.

$^1$H NMR δ: 0.81(s, 3H), 0.98(s, 3H), 1.02(t, J=7.4Hz, 6H), 1.08(d, J=6.9Hz, 3H), 1.63(g, J=7.4Hz, 2H), 1.64 (q, J=7.4Hz, 2H), 2.50-2.61(m, 1H), 2.72-2.87(m, 1H), 3.73-3.83(br. 1H), 4.00-4.15(m, 1H), 5.39(brs, 1H), 5.41-5.50 (m, 1H), 5.70-5.79(m, 1H).

(Example 6) Preparation of 1α,3β-dihydroxy-20(S)-(5-ethyl-5-hydroxy-3-heptynyl)-9,10-secopregna-5,7,10(19), 16-tetraene

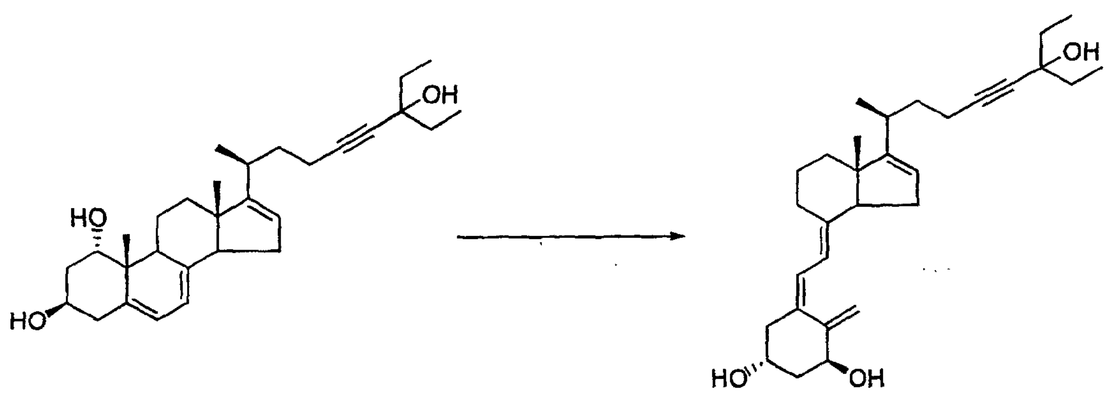

1α,3β-Dihydroxy-20(S)-(5-ethyl-5-hydroxy-3-heptynyl)pregna-5,7,16-triene (34.2 mg, 75.6 μmmol) was dissolved in ethanol (200 ml). While bubbling with argon at 0°C under stirring, the resulting solution was irradiated with a 400 W high-pressure mercury lamp through a Vycor filter for 3 minutes and 15 seconds, and then heated at reflux for 2 hours. After cooling to room temperature, the reaction mixture was evaporated under reduced pressure to remove the solvent and the resulting residue was purified by preparative thin layer chromatography (0.5 mm × 1 plate, hexane: ethyl acetate:ethanol = 10:10:1, developed twice; and then 0.25 mm × 1 plate, hexane:ethyl acetate:ethanol = 10:10: 0.8, developed three times) to give the titled compound (3.01 mg, 9%) as a colorless oil.

$^1$H NMR δ: 0.71(s, 3H), 0.98-1.09(m, 8H), 1.608(q, J=7.4Hz, 2H), 1.614(q, J=7.4Hz, 2H), 2.55-2.64(m, 1H),

2.78-2.87(m, 1H), 4.19-4.28(m, 1H), 4.39-4.48(m, 1H), 5.01(brs, 1H), 5.31-5.36(m, 2H), 6.10(d, J=11.2Hz, 1H), 6.38 (d, J=11.2Hz, 1H). IR(neat): 3388, 2929, 2456, 1373, 1144, 1055 $cm^{-1}$. MS m/z: 434($M^+$-$H_2O$), 105(100%). UV λmax nm: 263.

(Example 7) Preparation of 20(S)-1α,3β-bis(t-butyldimethylsilyloxy)-23-cyano-24-norchola-5,7,16-triene

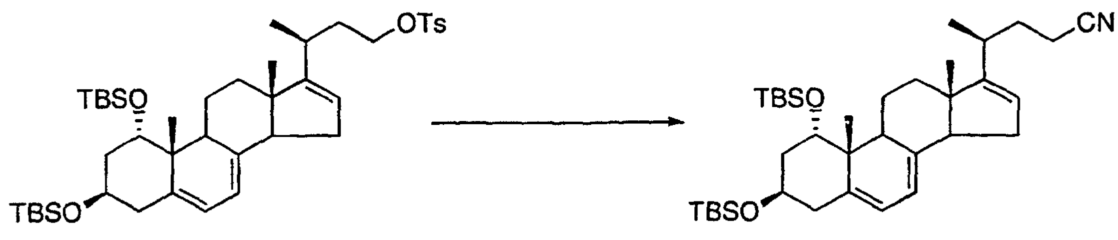

To a solution of 20(S)-1α,3β-bis(t-butyldimethylsilyloxy)-23-(p-toluenesulfonyloxy)-24-norchola-5,7,16-triene (816 mg, 1.10 mmol) in N,N-dimethylformamide (15 ml), potassium cyanide (573 mg, 8.80 mmol) was added and stirred at 65°C for 2 hours. After cooling to room temperature, the reaction mixture was poured into water and extracted with ethyl acetate. The extracted solution was washed with water and saturated brine, and then dried over anhydrous magnesium sulfate. After evaporation under reduced pressure to remove the solvent, the resulting residue was purified by silica gel column chromatography (hexane:ethyl acetate = 19:1) to give the titled compound (641 mg, 98%) as a white foam.

$^1H$ NMR δ: 0.06(s, 3H), 0.07(s, 3H), 0.08(s, 3H), 0.12(s, 3H), 0.81(s, 3H), 0.886(s, 9H), 0.889(s, 9H), 0.94(s, 3H), 1.11(d, J=6.9Hz, 3H), 2.80-2.92(m, 1H), 3.69-3.72(m, 1H), 3.98-4.12(m, 1H), 5.36-5.45(m, 2H), 5.58-5.64(m, 1H).

(Example 8) Preparation of 20(S)-1α,3β-bis(t-butyldimethylsilyloxy)chola-5,7,16-trien-24-al

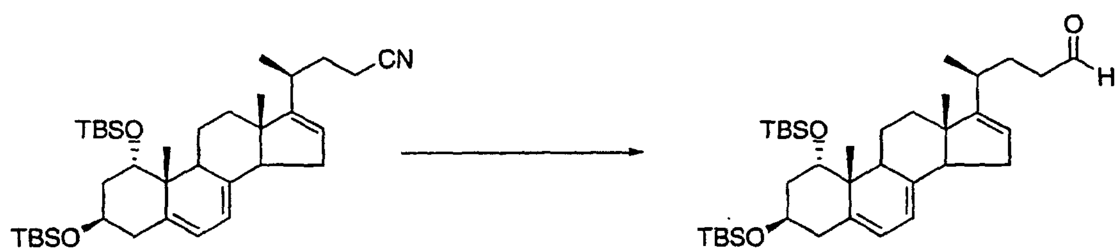

To a solution of 20(S)-1α,3β-bis(t-butyldimethylsilyloxy)-23-cyano-24-norchola-5,7,16-triene (417 mg, 0.700 mmol) in dichloromethane (12 ml), a 1.01 M toluene solution of diisobutylaluminum hydride (2.43 ml, 2.45 mmol) was added dropwise at -78°C and stirred at the same temperature for 2.5 hours. Hydrochloric acid (0.5 M) was poured into the reaction mixture, which was then extracted with ethyl acetate. The extracted solution was washed with saturated aqueous sodium bicarbonate and saturated brine, and then dried over anhydrous magnesium sulfate. After evaporation under reduced pressure to remove the solvent, the resulting residue was purified by silica gel column chromatography (hexane:ethyl acetate = 30:1) to give the titled compound (209 mg, 50%) as a colorless oil.

$^1H$ NMR δ: 0.06(s, 3H), 0.068(s, 3H), 0.073(s, 3H), 0.11(s, 3H), 0.80(s, 3H), 0.89(s, 18H), 0.94(s, 3H), 1.08 (d, J=6.8Hz, 3H), 2.70-2.80(m, 1H), 3.68-3.73(m, 1H), 3.98-4.12(m, 1H), 5.36-5.44(m, 2H), 5.59-5.64(m, 1H), 9.77(br, 1H). IR(neat): 2956, 2856, 1728, 1462, 1373, 1255, 1097 $cm^{-1}$. MS m/z: 598($M^+$), 409(100%). UV λmax nm: 270, 282, 294.

(Example 9) Preparation of 1α,3β-bis(t-butyldimethylsilyloxy)-20(S)-(3-ethyl-3-hydroxypentyl)pregna-5,7,16-triene

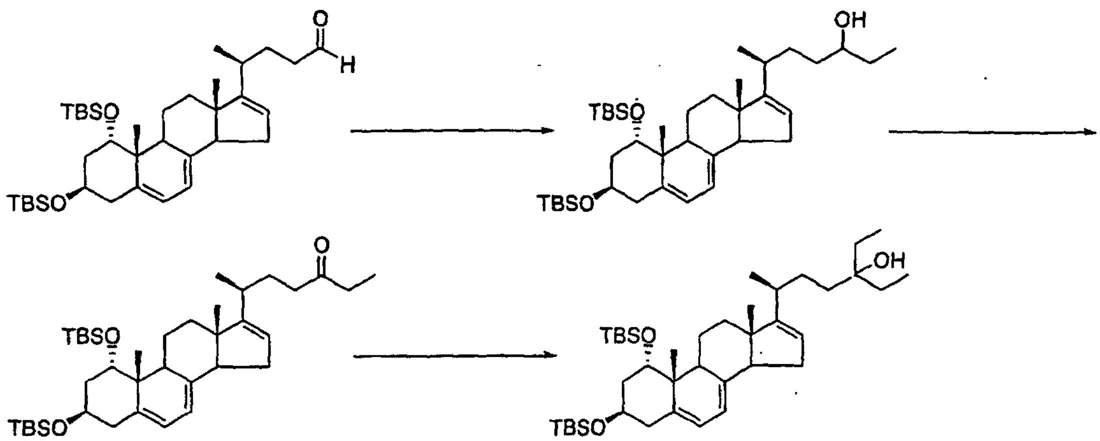

To a solution of 20(S)-1α,3β-bis(t-butyldimethylsilyloxy)chola-5,7,16-trien-24-al (158 mg, 264 μmol) in tetrahydrofuran (2 ml), a 1.00 M tetrahydrofuran solution of ethylmagnesium bromide (0.53 ml, 530 μmol) was added dropwise at 0°C and stirred at room temperature for 1 hour. Saturated aqueous ammonium chloride was poured into the reaction mixture, which was then extracted with ethyl acetate. The extracted solution was washed with saturated brine and dried over anhydrous magnesium sulfate. After evaporation under reduced pressure to remove the solvent, the resulting residue was purified by preparative thin layer chromatography (0.5 mm × 3 plates, hexane:ethyl acetate = 4:1, developed once) to give 1α,3β-bis(t-butyldimethyisilyloxy)-20(S)-(3-hydroxypentyl)pregna-5,7,16-triene (150 mg, 90%) as a colorless oil. This oil was dissolved in dichloromethane (2 ml) and mixed with 4-methylmorpholine-N-oxide (42 mg, 35.7 μmol) and molecular sieve 4A powder (500 mg), followed by stirring at room temperature for 10 minutes. To this mixture, tetra-n-propylammonium perruthenate (4.2 mg, 11.9 μmol) was added and stirred at room temperature for 1.5 hours. After insoluble products were filtered through Celite, the filtrate was washed with semi-saturated aqueous sodium sulfite and dried over anhydrous magnesium sulfate. After evaporation under reduced pressure to remove the solvent, the resulting residue was purified by preparative thin layer chromatography (0.5 mm × 3 plates, hexane:ethyl acetate = 9:1, developed once) to give 1α,3β-bis(t-butyldimethylsilyloxy)-20(S)-(3-oxopentyl)pregna-5,7,16-triene (102 mg, 68%) as a colorless oil. This oil was dissolved in tetrahydrofuran (2 ml) and mixed with a 1.00 M tetrahydrofuran solution of ethylmagnesium bromide (0.32 ml, 320 μmol) at 0°C, followed by stirring at room temperature for 1 hour. Saturated aqueous ammonium chloride was poured into the reaction mixture, which was then extracted with ethyl acetate. The extracted solution was washed with saturated brine and dried over anhydrous magnesium sulfate. After evaporation under reduced pressure to remove the solvent, the resulting residue was purified by preparative thin layer chromatography (0.5 mm × 2 plates, hexane:ethyl acetate = 4:1, developed once) to give the titled compound (73.0 mg, 69%) as a colorless oil.

$^1H$ NMR δ: 0.05(s, 3H), 0.07(s, 6H), 0.11(s, 3H), 0.80(s, 3H), 0.84(t, J=7.8Hz, 6H), 0.88(s, 9H), 0.89(s, 9H), 0.94(s, 3H), 1.07(d, J=6.7Hz, 3H), 1.45(q, J=7 .8Hz, 4H), 2.80-2.90(m, 1H), 3.68-3.73(m, 1H), 3.98-4.12(m, 1H), 5.35-5.42(m, 2H), 5.59-5.64(m, 1H). IR(neat): 2956, 2856, 1462, 1373, 1254, 1099 $cm^{-1}$. MS m/z: 656($M^+$), 467(100%). UV $\lambda_{max}$ nm: 270, 281, 293.

(Example 10) Preparation of 1α3β-dihydroxy-20(S)-(3-ethyl-3-hydroxypentyl)pregna-5,7,16-triene

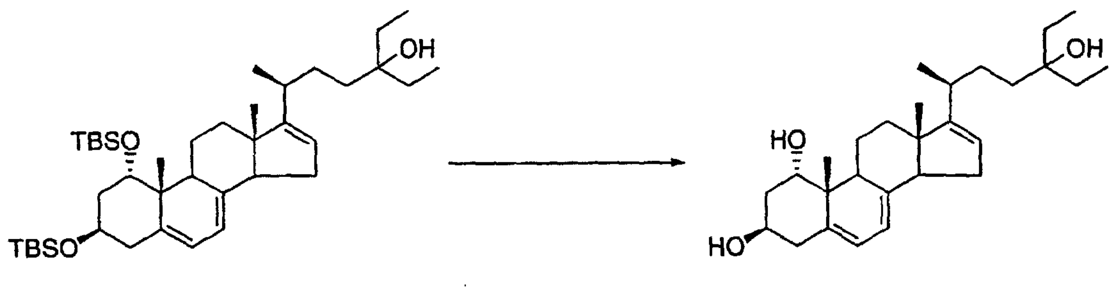

To a solution of 1α,3β-bis(t-butyldimethylsilyloxy)-20(S)-(3-ethyl-3-hydroxypentyl)pregna-5,7,16-triene (64.0 mg, 97.4 μmol) in tetrahydrofuran (2 ml), a 1M tetrahydrofuran solution of tetra-n-butylammonium fluoride (0.97 ml, 970 μmol) was added and stirred at 60°C for 5.5 hours. After cooling to room temperature, the reaction mixture was poured into water and extracted with ethyl acetate. The extracted solution was washed with saturated brine and dried over anhydrous magnesium sulfate. After evaporation under reduced pressure to remove the solvent, the resulting residue was purified by preparative thin layer chromatography (0.5 mm × 2 plates, dichloromethane:ethanol = 9:1; developed once) to give the titled compound (44.6 mg, quant.) as a white solid.

$^1H$ NMR δ: 0.79(s, 3H), 0.84(t, J=7.5Hz, 3H), 0.85(t, J=7.5Hz, 3H), 0.98(s, 3H), 1.08(d, J=6.9Hz, 3H), 1.45 (q, J=7.5Hz, 4H), 2.49-2.60(m, 1H), 2.72-2.86(m, 1H), 3.74-3.82(br, 1H), 4.00-4.16(m, 1H), 5.37(brs, 1H), 5.40-5.49 (m, 1H), 5.69-5.77(m, 1H). IR(neat): 3348, 2969, 1458, 1367, 1147, 1057 $cm^{-1}$. MS m/z: 428($M^+$), 285(100%). UV $\lambda_{max}$ nm: 270, 281, 293.

(Example 11) Preparation of 1α,3β-dihydroxy-20(S)-(3-ethyl-3-hydroxypentyl)-9,10-secopregna-5,7,10(19), 16-tetraene

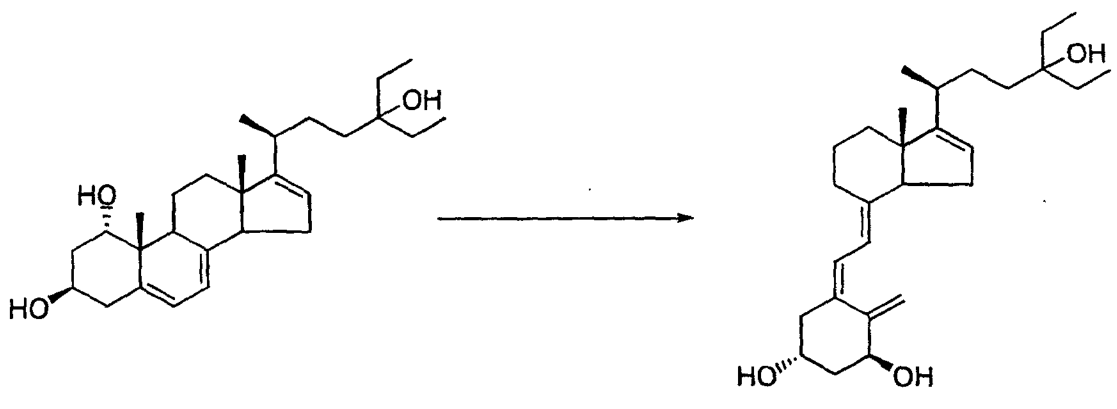

1α,3β-Dihydroxy-20(S)-(3-ethyl-3-hydroxypentyl)pregna-5,7,16-triene (41.6 mg, 97.0 μmol) was dissolved in ethanol (200 ml). While bubbling with argon at 0°C under stirring, the resulting solution was irradiated with a 400 W high-pressure mercury lamp through a Vycor filter for 4 minutes, and then heated at reflux for 2 hours. After cooling to room temperature, the reaction mixture was evaporated under reduced pressure to remove the solvent and the resulting residue was purified by preparative thin layer chromatography (0.5 mm × 2 plates. dichloromethane:ethanol = 9:1, developed twice; and then 0.5 mm × 1 plate, hexane:ethyl acetate:ethanol = 5:5:0.3, developed three times) to give the titled compound (3.51 mg, 8%) as a colorless oil.

$^1H$ NMR δ: 0.70(s, 3H), 0.85(t, J=7.5Hz, 6H), 1.06(d, J=6.9Hz, 3H), 1.45(q, J=7.5Hz, 4H), 2.55-2.65(m, 1H), 2.76-2.88(m, 1H), 4.19-4.30(m, 1H), 4.40-4.50(m, 1H), 5.01(brs, 1H), 5.28-5.38(m, 2H), 6.11(d, J=11.2Hz, 1H), 6.38(d, J=11.2Hz, 1H). IR(neat): 3367, 2931, 1456, 1373, 1288, 1146. 1055 $cm^{-1}$. MS m/z: 428($M^+$). 134(100%). UV $\lambda_{max}$nm: 263.

(Example 12) Preparation of 20(R)-1α3β-bis (t-butyldimethylsilyloxy)-24-norchola-5,7,16-trien-23-al

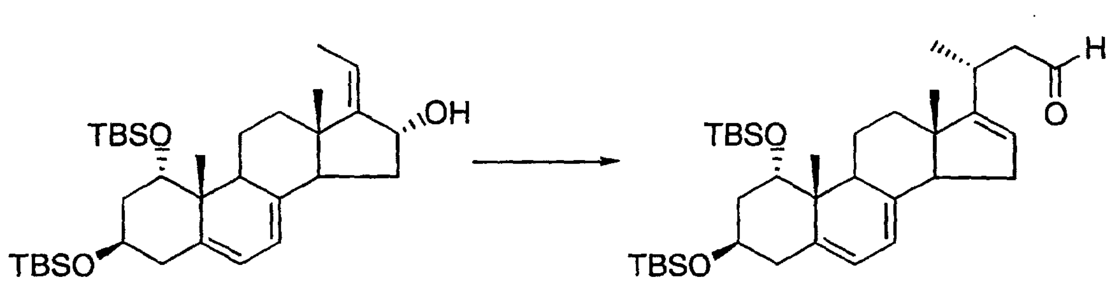

1α,3β-Bis(t-butyldimethylsilyloxy)-16α-hydroxypregna-5,7,17(E)-triene (511 mg, 0.914 mmol) was dissolved in ethyl vinyl ether (11 ml), followed by addition of mercury(II) acetate (233 mg, 0.731 mmol) and stirring at room temperature for 40 hours. The reaction mixture was poured into 5% aqueous potassium hydroxide and extracted with hexane. The organic layer was washed with water and saturated brine, dried over anhydrous sodium sulfate, and then evaporated under reduced pressure to remove the solvent. The resulting residue was dissolved in 1,3-dimethyl-2-imidazolidinone (15 ml) and stirred at 110°C for 3 hours. After cooling to room temperature, the reaction mixture was poured into water and extracted with ethyl acetate. The organic layer was washed with water and saturated brine, dried over anhydrous sodium sulfate, and then evaporated under reduced pressure to remove the solvent. The resulting residue was purified by silica gel column chromatography (hexane:ethyl acetate = 5:1) to give the titled compound (398 mg, 74%) as a white solid.

$^1H$ NMR δ: 0.06(s, 3H), 0.067(s, 3H), 0.074(s, 3H), 0.12(s, 3H), 0.81(s, 3H), 0.89(s, 18H), 0.94(s, 3H), 1.12 (d, J=6.6Hz, 3H), 3.70-3.75(m, 1H), 3.97-4.12(m, 1H), 5.34-5.42(m, 2H), 5.57-5.64(m, 1H), 9.68(t, J=2.1Hz, 1H).

(Example 13) Preparation of 20(R)-1α3β-bis(t-butyldimethylsilyloxy)-24-ethoxycarbonylchola-5,7,16,23(E)-tetraene

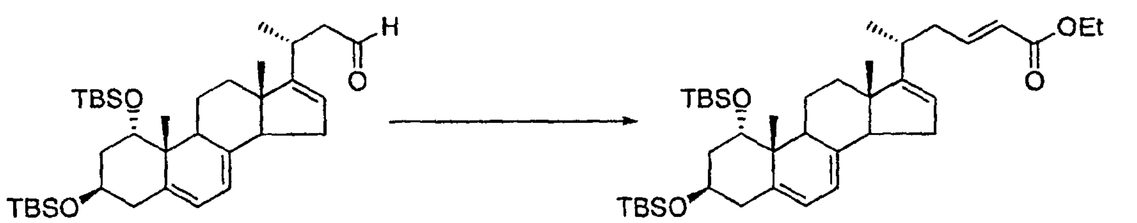

To a mixture of lithium bromide (22 mg, 0.253 mmol) in tetrahydrofuran (0.5 ml), triethyl phosphonoacetate (52 μl, 0.260 mmol) was added and stirred at room temperature for 5 minutes, followed by addition of triethylamine (36 μl, 0.258 mmol) and stirring at room temperature for 10 minutes. To this reaction mixture, a solution of 20(R)-1α, 3β-bis(t-butyldimethylsilyloxy)-24-norchola-5,7,16-trien-23-al (100 mg, 0.171 mmol) in tetrahydrofuran (0.5 ml) was added and stirred at room temperature for 3 hours. The reaction mixture was diluted with ethyl acetate, washed with ice-cold 0.5 M hydrochloric acid, saturated aqueous sodium bicarbonate and saturated brine, and then dried over anhydrous sodium sulfate. After evaporation under reduced pressure to remove the solvent, the resulting residue was purified by silica gel column chromatography (hexane:ethyl acetate = 10:1) to give the titled compound (113 mg, quant.) as a colorless oil.

$^1H$ NMR δ: 0.06(s, 3H), 0.067(s, 3H), 0.072(s, 3H), 0.11(s, 3H), 0.79(s, 3H), 0.89(s, 18H), 0.94(s, 3H), 1.06 (d, J=6.3Hz, 3H), 1.28(t, J=7.2Hz, 3H), 2.81-2.91(m, 1H), 3.67-3.75(m, 1H), 3.98-4.12(m, 1H), 4.18(q, J=7.2Hz, 2H), 5.35-5.43(m, 2H), 5.57-5.65(m, 1H), 5.81(d, J=15.5Hz, 1H), 6.90(dt, J=15.5, 7.6Hz, 1H). MS m/z: 654 ($M^+$), 465(100%).

(Example 14) Preparation of 20(R)-1α-3β-bis(t-butyldimethylsilyloxy)-24-ethoxycarbonylchola-5,7,16-triene

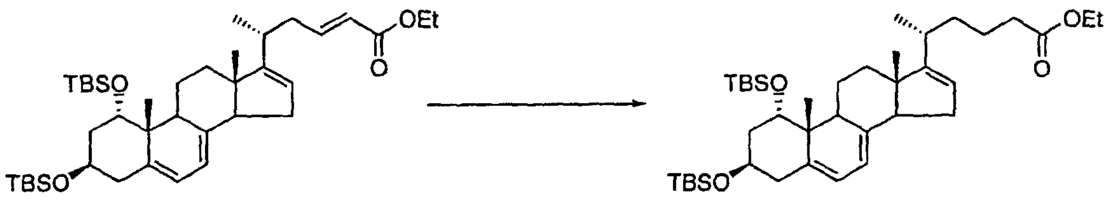

20(R)-1α,3β-Bis(t-butyldimethylsilyloxy)-24-ethoxycarbonylchola-5,7,16,23(E)-tetraene (97.6 mg, 0.149 mmol), nickel(II) chloride hexahydrate (4 mg, 16.8 μmol), tetrahydrofuran (1 ml) and methanol (3 ml) were mixed and cooled to 0°C, followed by addition of sodium borohydride (17 mg, 0.449 mmol). After stirring at room temperature for 30 minutes, water and ethyl acetate were added to the reaction mixture and insoluble products were filtered off. The organic layer was washed with saturated brine and dried over anhydrous sodium sulfate. After evaporation under reduced pressure to remove the solvent, the resulting residue was purified by silica gel column chromatography (hexane:ethyl acetate = 10:1) to give the titled compound (87.6 mg, 89%) as a colorless oil.

$^1H$ NMR δ: 0.06(s, 3H), 0.07(s, 6H), 0.11(s, 3H), 0.78(s, 3H), 0.89(s, 18H), 0.94(s, 3H), 1.03(d, J=6.9Hz, 3H), 1.25(t, J=7.2Hz, 3H), 2.80-2.91(m, 1H), 3.68-3.74(m, 1H), 3.98-4.12(m, 1H), 4.12(q, J=7.2Hz, 2H), 5.31-5.43(m, 2H), 5.55-5.64(m, 1H). MS m/z: 656($M^+$), 467(100%).

(Example 15) Preparation of 20(R)-1α,3β-bis(t-butyldimethylsilyloxy)-25-hydroxycholesta-5,7,16-triene

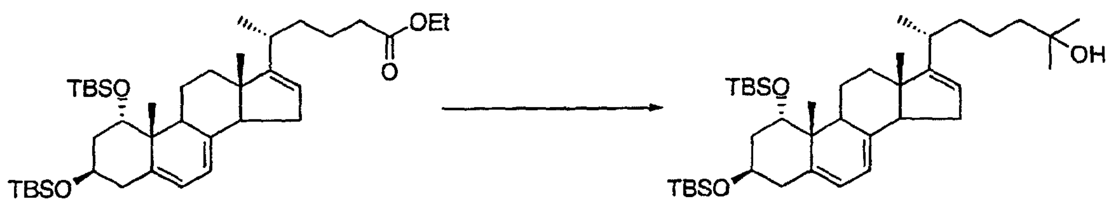

To a solution of 20(R)-1α,3β-bis(t-butyldimethylsilyloxy)-24-ethoxycarbonylchola-5,7,16-triene (45.5 mg, 69.2 μmol) in tetrahydrofuran (1.5 ml), a 0.92 M tetrahydrofuran solution of methylmagnesium bromide (0.8 ml, 736 μmol) was added dropwise at room temperature and stirred at room temperature for 1 hour. After cooling to 0°C, saturated aqueous ammonium chloride was poured into the reaction mixture, which was then extracted with ethyl acetate. The extracted solution was washed with saturated brine and dried over anhydrous sodium sulfate. After evaporation under reduced pressure to remove the solvent, the resulting residue was purified by silica gel column chromatography (hexane:ethyl acetate = 2:1) to give the titled compound (37.1 mg, 83%) as a colorless oil.

$^1H$ NMR δ: 0.05(s, 3H), 0.07(s, 6H), 0.11(s, 3H), 0.79(s, 3H), 0.88(s, 18H), 0.94(s, 3H), 1.03(d, J=6.6Hz, 3H), 1.20(s, 6H), 2.80-2.91(m, 1H), 3.67-3.74(m, 1H), 3.98-4.12(m, 1H), 5.30-5.42(m, 2H), 5.57-5.64(m, 1H). MS m/z: 642 ($M^+$). 453(100%).

(Example 16) Preparation of 20(R)-1α,3β,25-trihydroxycholesta-5,7,16-triene

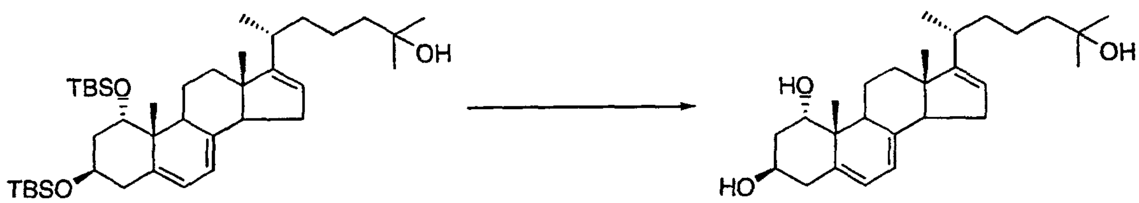

To a solution of 20(R)-1α,3β-bis(t-butyldimethylsilyloxy)-25-hydroxycholesta-5,7,16-triene (22.0 mg, 34.2 μmol) in tetrahydrofuran (3 ml), a 1M tetrahydrofuran solution of tetra-n-butylammonium fluoride (0.51 ml, 510 μmol) was added, followed by heating at reflux for 3.5 hours. After cooling to room temperature, the reaction mixture was diluted with ethyl acetate, washed with saturated aqueous sodium bicarbonate and saturated brine, and then dried over anhydrous sodium sulfate. After evaporation under reduced pressure to remove the solvent, the resulting residue was purified by silica gel column chromatography (chloroform:methanol = 10:1) to give the titled compound (15.8 mg, quant.) as a white solid.

$^1$H NMR δ: 0.80(s, 3H), 0.98(s, 3H), 1.03(d, J=6.6Hz, 3H), 1.20(s, 6H), 2.49-2.61(m, 1H), 2.74-2.85(m, 1H), 3.75-3.83(m, 1H), 3.98-4.16(m, 1H), 5.31-5.37(br, 1H), 5.42-5.49(m, 1H), 5.71-5.79(m, 1H).

(Example 17) Preparation of 20(R)-1α,3β,25-trihydroxy-9,10-secocholesta-5,7,10(19) ,16-tetraene

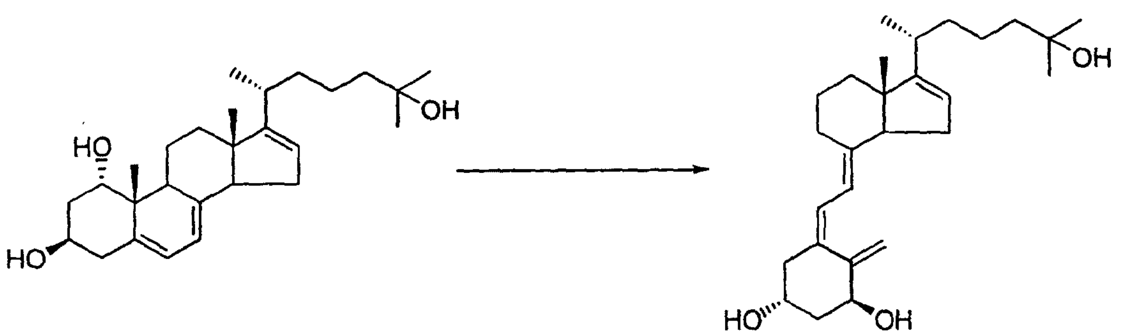

20(R)-1α,3β,25-Trihydroxycholesta-5,7,16-triene (36.4 mg, 87.8 μmol) was dissolved in ethanol (200 ml). While bubbling with argon at 0°C under stirring, the resulting solution was irradiated with a 400 W high-pressure mercury lamp through a Vycor filter for 3 minutes and 15 seconds, and then heated at reflux for 2 hours. After cooling to room temperature, the reaction mixture was evaporated under reduced pressure to remove the solvent and the resulting residue was purified by preparative thin layer chromatography (0.5 mm × 1 plate, hexane:ethyl acetate: ethanol = 10: 10:1, developed twice; and then 0.25 mm × 1 plate, dichloromethane:ethyl acetate:ethanol = 7:3:0.5, developed three times) to give the titled compound (4.30 mg, 12%) as a colorless oil.

$^1$H NMR δ: 0.69(s, 3H), 1.03(d, J=6.7Hz, 3H), 1.20(s, 6H), 2.57-2.65(m, 1H), 2.76-2.88(m, 1H), 4.17-4.29(m, 1H), 4.39-4.49(m, 1H), 5.02(brs, 1H), 5.29(brs, 1H), 5.34(brs, 1H), 6.11(d, J=11.2Hz, 1H), 6.38(d, J=11.2Hz, 1H). IR (neat): 3367, 2964, 1450, 1367, 1207, 1151, 1054 $cm^{-1}$. MS m/z: 414($M^+$), 134(100%). UV $\lambda_{max}$ nm: 263.

(Example 18) Preparation of 20(R)-1α,3β-bis(t-butyldimethylsilyloxy)-25-hydroxy-26,27-bishomocholesta-5,7,16-triene

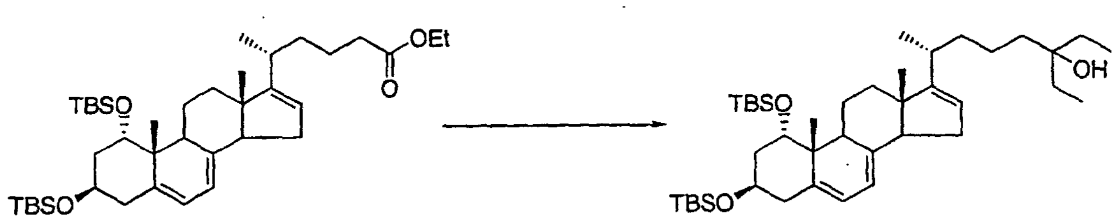

To a solution of 20(R)-1α,3β-bis(t-butyldimethylsilyloxy)-24-ethoxycarbonylchola-5,7,16-triene (30.0 mg, 45.7 μmol) in tetrahydrofuran (1.5 ml), a 1.00 M tetrahydrofuran solution of ethylmagnesium bromide (0.46 ml, 460 μmol) was added dropwise at room temperature and stirred at room temperature for 1 hour. After cooling to 0°C, saturated aqueous ammonium chloride was poured into the reaction mixture, which was then extracted with ethyl acetate. The extracted solution was washed with saturated brine and dried over anhydrous sodium sulfate. After evaporation under reduced pressure to remove the solvent, the resulting residue was purified by silica gel column chromatography (hexane:ethyl acetate = 4:1) to give the titled compound (30.4 mg, 99%) as a colorless oil.

$^1$H NMR δ: 0.05(s, 3H), 0.066(s, 3H), 0.070(s, 3H), 0.11(s, 3H), 0.79(s, 3H), 0.85(t, J=7.6Hz, 6H), 0.88(s, 18H), 0.94(s, 3H), 1.03(d, J=6.9Hz, 3H), 1.45(q, J=7.6Hz, 4H), 2.78-2.92(m, 1H), 3.68-3.75(br, 1H), 3.98-4.12(m, 1H), 5.33(br, 1H), 5.34-5.42(m, 1H), 5.56-5.65(m, 1H). MS m/z: 670($M^+$), 481(100%).

(Example 19) Preparation of 20(R)-1α,3β,25-trihydroxy-26, 27-bishomocholesta-5,7,16-triene

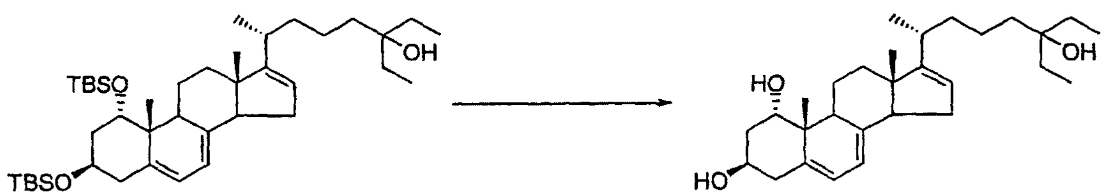

To a solution of 20(R)-1α,3β-bis(t-butyldimethylsilyloxy)-25-hydroxy-26,27-bishomocholesta-5,7,16-triene (17.5 mg, 26.1 μmol) in tetrahydrofuran (3 ml), a 1M tetrahydrofuran solution of tetra-n-butylammonium fluoride (0.32 ml, 320 μmol) was added, followed by heating at reflux for 3.5 hours. After cooling to room temperature, the reaction mixture was diluted with ethyl acetate, washed with saturated aqueous sodium bicarbonate and saturated brine, and then dried over anhydrous sodium sulfate. After evaporation under reduced pressure to remove the solvent, the resulting residue was purified by silica gel column chromatography (chloroform:methanol = 10:1) to give the titled compound (13.6 mg, quant.) as a colorless foam.

$^1$H NMR δ: 0.80(s, 3H), 0.85(t, J=7.5Hz, 6H), 0.98(s, 3H), 1.02(d, J=6.9Hz, 3H), 1.44(q, J=7.5Hz, 4H), 2.49-2.61(m, 1H), 2.73-2.85(m, 1H), 3.72-3.83(br, 1H), 3.98-4.15(m, 1H), 5.33(brs, 1H), 5.42-5.51(m, 1H), 5.61-5.69 (m, 1H).

(Example 20) Preparation of 20(R)-1α,3β,25-trihydroxy-26.27-bishomo-9,10-secocholesta-5,7,10(19),16-tetraene

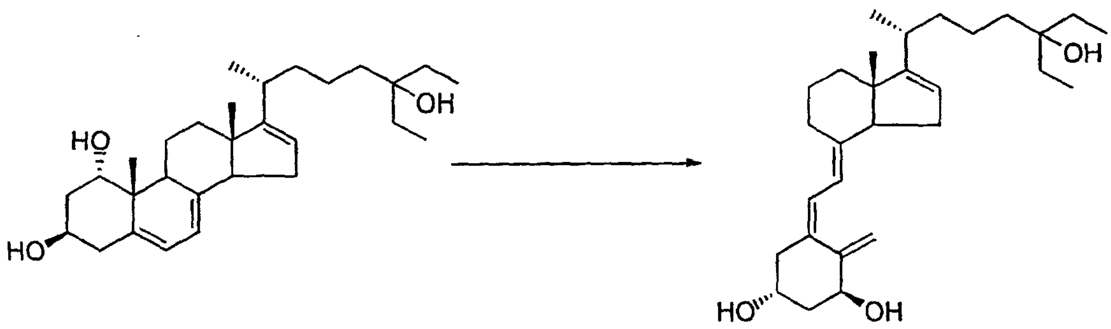

20(R)-1α,3β,25-Trihydroxy-26,27-bishomocholesta-5,7,16-triene (45.5 mg, 103 μmol) was dissolved in ethanol (200 ml). While bubbling with argon at 0°C under stirring, the resulting solution was irradiated with a 400 W high-pressure mercury lamp through a Vycor filter for 3 minutes and 45 seconds, and then heated at reflux for 2 hours. After cooling to room temperature, the reaction mixture was evaporated under reduced pressure to remove the solvent and the resulting residue was purified by preparative thin layer chromatography (0.5 mm × 1 plate, hexane:ethyl acetate: ethanol = 10:10:1, developed twice; and then 0.25 mm × 1 plate, dichloromethane:ethyl acetate:ethanol = 7:3:0.5, developed three times) to give the titled compound (5.76 mg, 13%) as a colorless oil.

$^1$H NMR δ: 0.69(s, 3H), 0.85(t, J=7.5Hz, 6H), 1.02(d, J=6.9Hz, 3H), 1.44(q, J=7.5Hz, 4H), 2.54-2. 66(m, 1H), 2.76-2.88(m, 1H), 4.17-4.30(br, 1H), 4.38-4.50(br, 1H), 5.02(brs, 1H), 5.29(brs, 1H), 5.34(brs, 1H), 6.11(d, J=11.3Hz, 1H), 6.38(d, J=11.3Hz, 1H). IR(neat): 3381, 2962, 1460, 1369, 1146, 1055 $cm^{-1}$. MS m/z: 442($M^+$), 134(100%). UV $\lambda_{max}$ nm: 263.

(Example 21) Preparation of 20(S)-1α,3β-bis(t-butyldimethylsilyloxy-24-ethoxycarbonylchola-5,7,16,23(E)-tetraene

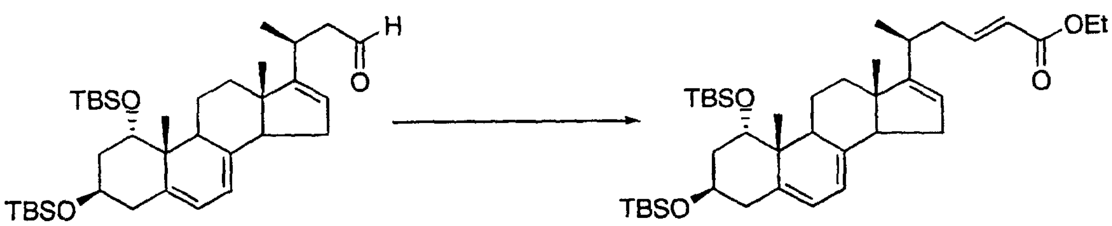

To a mixture of lithium bromide (61.3 mg, 0.707 mmol) in tetrahydrofuran (3 ml), triethyl phosphonoacetate (113 μl, 0.566 mmol) was added at 0°C and stirred at 0°C for 5 minutes, followed by addition of triethylamine (98.9 μl, 0.707 mmol) and stirring at 0°C for 10 minutes. To this reaction mixture, a solution of 20(S)-1α,3β-bis(t-butyldimethylsilyloxy)-24-norchola-5,7,16-trien-23-al (276 mg, 0.471 mmol) in tetrahydrofuran (3 ml) was added and stirred at room temperature for 3 hours. Water was added to the reaction mixture, which was then extracted with ethyl acetate. The extracted solution was washed with ice-cold 0.5 M hydrochloric acid, saturated aqueous sodium bicarbonate and saturated brine, and then dried over anhydrous magnesium sulfate. After evaporation under reduced pressure to remove the solvent, the resulting residue was purified by silica gel column chromatography (hexane:ethyl acetate = 25:1) to give the titled compound (301 mg, 98%) as a colorless oil.

$^1$H NMR δ: 0.06(s, 3H), 0.07(s, 3H), 0.08(s, 3H), 0.12(s, 3H), 0.81(s, 3H), 0.89(s, 18H), 0.95(s, 3H), 1.06(d, J=5.6Hz, 3H), 1.28(t, J=7.2Hz, 3H), 2.80-2.92(m, 1H), 3.68-3.76(br, 1H), 3.98-4.12(m, 1H), 4.18(q, J=7.2Hz, 2H), 5.35-5.45(m, 2H), 5.66-5.75(m, 1H), 5.81(d, J=15.2Hz, 1H), 6.92(dt, J=15.2, 7.5Hz, 1H).

(Example 22) Preparation of 20(S)-1α,3β-bis(t-butyldimethylsilyloxy-24-ethoxycarbonylchola-5,7,16-triene

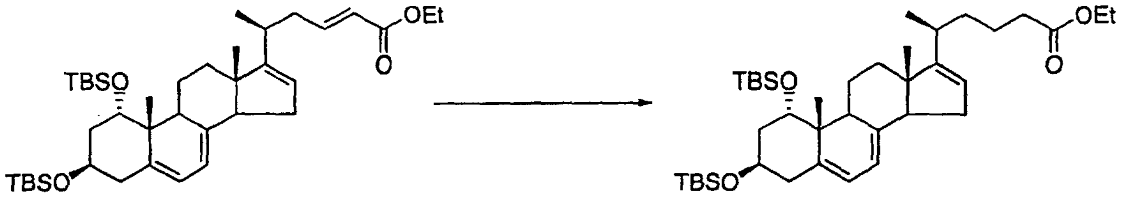

20(S)-1α,3β-Bis(t-butyldimethylsilyloxy)-24-ethoxycarbonylchola-5,7,16,23(E)-tetraene (205 mg, 0.312 mmol), nickel(II) chloride hexahydrate (14.8 mg, 62 μmol), tetrahydrofuran (2 ml) and methanol (6 ml) were mixed and cooled to 0°C, followed by addition of sodium borohydride (39.4 mg, 0.936 mmol). After stirring at room temperature for 30 minutes, water and ethyl acetate were added to the reaction mixture and insoluble products were filtered off. The organic layer was washed with saturated brine and dried over anhydrous magnesium sulfate. After evaporation under reduced pressure to remove the solvent, the resulting residue was purified by silica gel column chromatography (hexane:ethyl acetate = 15:1) to give the titled compound (197 mg, 96%) as a colorless oil.

$^1$H NMR δ: 0.06(s, 3H), 0.067(s, 3H), 0.074(s, 3H), 0.11(s. 3H), 0.80(s, 3H), 0.89(s, 18H), 0.94(s, 3H), 1.07 (d, J=6.9Hz, 3H), 1.25(t, J=7.1Hz, 3H), 2.79-2.91(m, 1H), 3.67-3.75(br, 1H), 3.98-4.14(m, 1H), 4.12(q, J=7.1Hz, 2H), 5.31-5.42(m, 2H), 5.55-5.63(m, 1H).

(Example 23) Preparation of 20(S)-1α,3β-bis(t-butyldimethylsilyloxy)-25-hydroxycholesta-5,7,16-triene

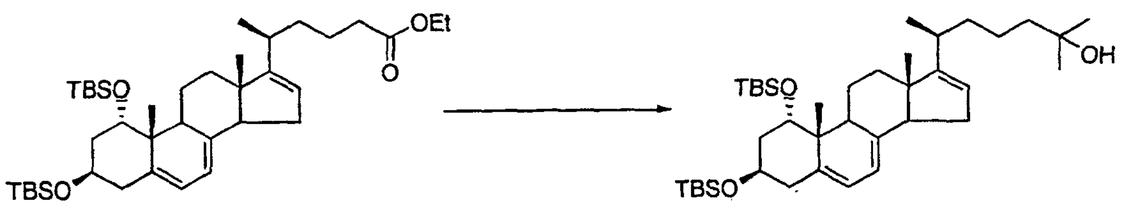

To a solution of 20(S)-1α,3β-bis(t-butyldimethylsilyloxy)-24-ethoxycarbonylchola-5,7,16-triene (115 mg, 0.175 mmol) in tetrahydrofuran (5 ml), a 0.92 M tetrahydrofuran solution of methylmagnesium bromide (1.91 ml, 1.76 mmol) was added dropwise at 0°C and stirred at 0°C for 30 minutes, followed by 1 hour at room temperature. After cooling to 0°C, ice-cold water was poured into the reaction mixture, which was then extracted with ethyl acetate. The extracted solution was washed with saturated brine and dried over anhydrous magnesium sulfate. After evaporation under reduced pressure to remove the solvent, the resulting residue was purified by silica gel column chromatography (hexane: ethyl acetate = 12:1) to give the titled compound (66.4 mg, 59%) as a colorless oil.

$^1$H NMR δ: 0.06(s, 3H), 0.067(s, 3H), 0.073(s, 3H), 0.11(s, 3H), 0.80(s, 3H), 0.89(s, 18H), 0.95(s, 3H), 1.06 (d, J=6.9Hz, 3H), 1.20(s, 6H), 2.78-2.91(m, 1H), 3.68-3.75(br, 1H), 3.98-4.12(m, 1H), 5.32-5.45(m, 2H), 5.56-5.78(m, 1H).

(Example 24) Preparation of 20(S)-1α,3β,25-trihydroxycholesta-5,7,16-triene

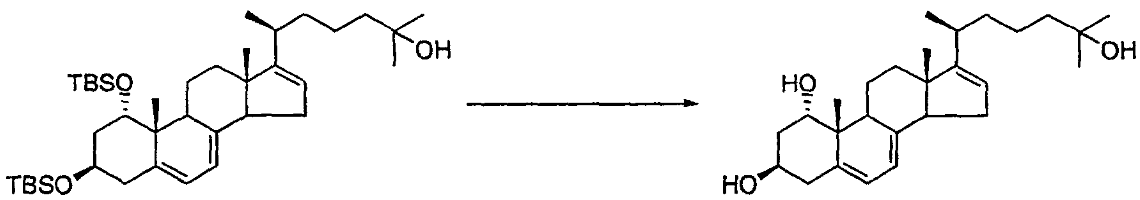

To a solution of 20(S)-1α,3β-bis(t-butyldimethylsilyloxy)-25-hydroxycholesta-5,7,16-triene (66.4 mg, 0.103 mmol) in tetrahydrofuran (2 ml), a 1M tetrahydrofuran solution of tetra-n-butylammonium fluoride (1.03 ml, 1.03 mmol) was added, followed by heating at reflux for 6 hours. After cooling to room temperature, the reaction mixture was diluted with ethyl acetate, washed with water and saturated brine, and then dried over anhydrous magnesium sulfate. After evaporation under reduced pressure to remove the solvent, the resulting residue was purified by silica gel column chromatography (chloroform:ethyl acetate = 2:1) to give the titled compound (35.0 mg, 82%) as a white solid.

$^1H$ NMR δ: 0.81(s, 3H), 0.99(s, 3H), 1.07(d, J=6.9Hz, 3H), 1.21(s, 6H), 2.50-2.61(m, 1H), 2.73-2.85(m, 1H), 3.75-3.83(br, 1H), 3.98-4.16(m, 1H), 5.31-5.37(br, 1H), 5.42-5.50(m, 1H), 5.71-5.79(m, 1H).

(Example 25) Preparation of 20(S)-1α,3β,25-trihydroxy-9,10-secocholesta-5,7,10(19),16-tetraene

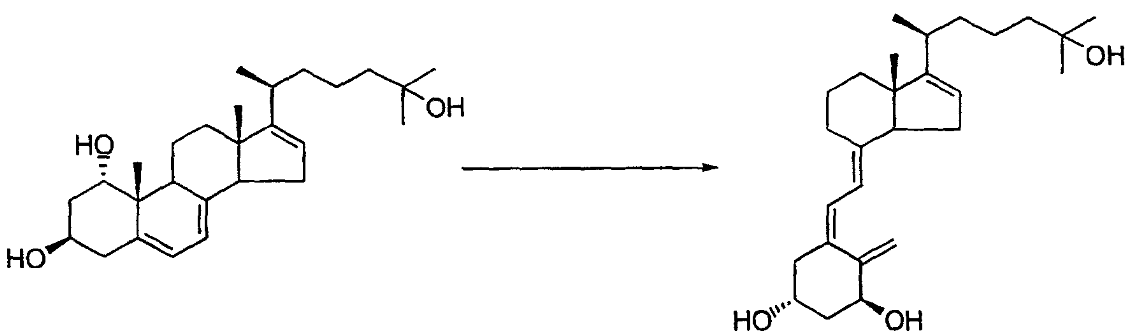

20(s)-1α,3β,25-Trihydroxycholesta-5,7,16-triene (32.2 mg, 77.7 μmol) was dissolved in ethanol (200 ml). While bubbling with argon at 0°C under stirring, the resulting solution was irradiated with a 400 W high-pressure mercury lamp through a Vycor filter for 3 minutes and 15 seconds, and then heated at reflux for 2 hours. After cooling to room temperature, the reaction mixture was evaporated under reduced pressure to remove the solvent and the resulting residue was purified by preparative thin layer chromatography (0.5 mm × 1 plate, hexane:ethyl acetate:ethanol = 10:10:1, developed three times; and then 0.25 mm × 1 plate, dichloromethane:ethyl acetate:ethanol = 7:3:0.5, developed twice) to give the titled compound (3.77 mg, 12%) as a colorless oil.

$^1H$ NMR δ: 0.70(s, 3H), 1.05(d, J=6.8Hz, 3H), 1.21(s, 6H), 2.55-2.66(m, 1H), 2.76-2.87(m, 1H), 4.19-4.30(m, 1H), 4.40-4.50(m, 1H), 5.01(brs. 1H), 5.30-5.39(m, 2H), 6.11(d, J=11.2Hz, 1H), 6.38(d, J=11.2Hz, 1H). IR(neat): 3365, 2931, 1464, 1367, 1207, 1153, 1055 $cm^{-1}$. MS m/z: 414($M^+$), 133(100%). UV $\lambda_{max}$ nm: 263.

(Example 26) Preparation of 20(S)-1α,3β-bis(t-butyldimethylsilyloxy-25-hydroxy-26,27-bishomocholesta-5,7,16-triene

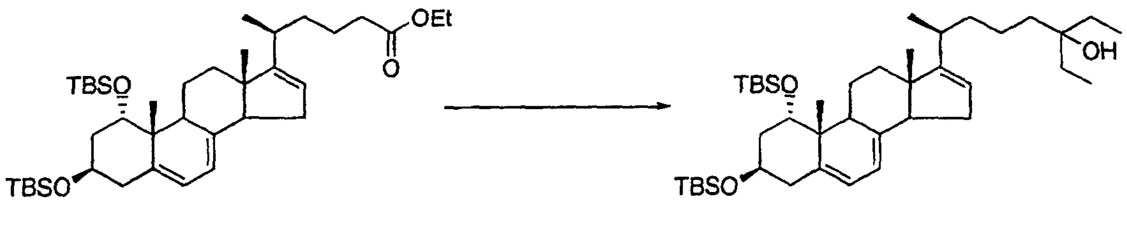

To a solution of 20(S)-1α,3β-bis(t-butyldimethylsilyloxy)-24-ethoxycarbonylchola-5,7,16-triene (82.1 mg, 125 μmol) in tetrahydrofuran (5 ml), a 1.04 M tetrahydrofuran solution of ethylmagnesium bromide (1.20 ml, 1.25 mmol) was added dropwise at room temperature and stirred at room temperature for 1 hour. The reaction mixture was poured into ice-cold water and extracted with ethyl acetate. The extracted solution was washed with saturated brine and dried over anhydrous magnesium sulfate. After evaporation under reduced pressure to remove the solvent, the resulting residue was purified by silica gel column chromatography (hexane:ethyl acetate = 12:1) to give the titled compound (63.5 mg, 76%) as a colorless oil.

$^1$H NMR δ: 0.06(s, 3H), 0.068(s, 3H), 0.073(s, 3H), 0.11(s, 3H), 0.80(s, 3H), 0.85(t, J=7.5Hz, 6H), 0.89(s, 18H), 0.95(s, 3H), 1.06(d, J=6.9Hz, 3H), 1.45(q, J=7.5Hz, 4H), 2.79-2.91(m, 1H), 3.67-3.76(br, 1H), 3.97-4.12(m, 1H), 5.28-5.44(m, 2H), 5.56-5.66(m, 1H).

(Example 27) Preparation of 20(S)-1α,3β,25-trihydroxy-26.27-bishomocholesta-5,7,16-triene

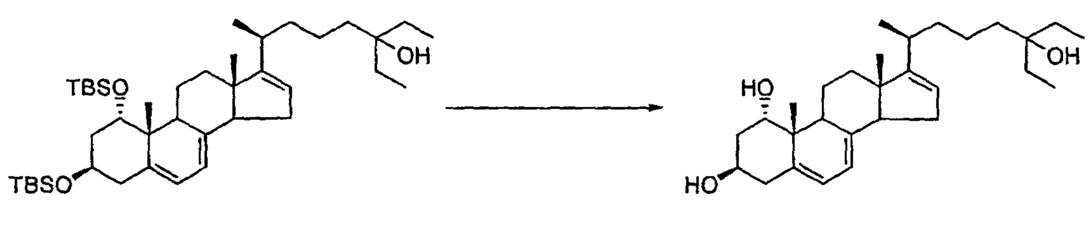

To a solution of 20(S)-1α,3β-bis(t-butyldimethylsilyloxy)-25-hydroxy-26,27-bishoniocholesta-5,7,16-triene (63.5 mg, 94.6 μmol) in tetrahydrofuran (2 ml), a 1M tetrahydrofuran solution of tetra-n-butylammonium fluoride (0.95 ml, 950 μmol) was added, followed by heating at reflux for 4 hours. After cooling to room temperature, the reaction mixture was diluted with ethyl acetate, washed with water and saturated brine, and then dried over anhydrous magnesium sulfate. After evaporation under reduced pressure to remove the solvent, the resulting residue was purified by silica gel column chromatography (hexane:ethyl acetate = 1:1 and then 1:3) to give the titled compound (39.6 mg, 94%) as a white solid.

$^1$H NMR δ: 0.81(s, 3H), 0.85(t, J=7.5Hz, 6H), 0.98(s, 3H), 1.07(d, J=6.9Hz, 3H), 1.45(q, J=7.5Hz, 4H), 2.49-2.61(m, 1H), 2.71-2.85(m, 1H), 3.74-3.83(br, 1H), 3.99-4.15(m, 1H), 5.36(brs, 1H), 5.41-5.50(m, 1H), 5.70-5.80 (m 1H).

(Example 28) Preparation of 20(S)-1α,3β,25-trihydroxy-26,27-bishomo-9,10-secocholesta-5,7,10(19),16-tetraene

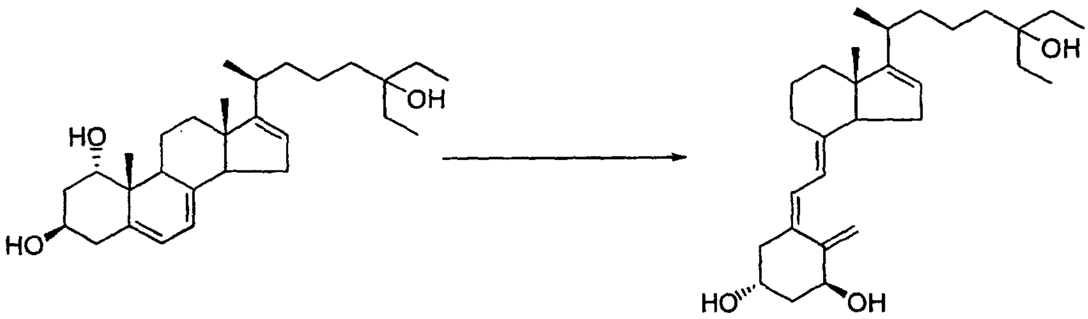

20(S)-1α,3β,25-Trihydroxy-26,27-bishomocholesta-5,7,16-triene (32.7 mg, 73.9 μmol) was dissolved in ethanol (200 ml). While bubbling with argon at 0°C under stirring, the resulting solution was irradiated with a 400 W high-pressure mercury lamp through a Vycor filter for 3 minutes and 15 seconds, and then heated at reflux for 2 hours. After cooling to room temperature, the reaction mixture was evaporated under reduced pressure to remove the solvent and the resulting residue was purified by preparative thin layer chromatography (0.5 mm × 1 plate, hexane:ethyl acetate:ethanol = 10:10:1, developed twice; and then 0.25 mm × 1 plate, dichloromethane:ethyl acetate:ethanol = 7:3:0.5, developed twice) to give the titled compound (4.00 mg, 12%) as a colorless oil.

$^1$H NMR δ: 0.70(s, 3H), 0.86(t, J=7.4Hz, 6H), 1.05(d, J=6.9Hz, 3H), 1.46(q, J=7.4Hz, 4H), 2.55-2.68(m, 1H), 2.76-2.89(m, 1H), 4.18-4.30(br, 1H), 4.39-4.50(br, 1H), 5.01(brs, 1H). 5.28-5.38(m, 2H), 6.11(d, J=11.2Hz, 1H), 6.38 (d, J=11.2Hz, 1H). IR(neat): 3373, 2933, 1458, 1373, 1146, 1055 $cm^{-1}$. MS m/z: 442($M^+$), 133(100%). UV $\lambda_{max}$ nm: 263.

(Test Example) Test in osteoporosis rat model

The compounds of the present invention were evaluated for their effects on bone mineral density and blood calcium (Ca) level in osteoporosis rat model. The following compounds were tested: 1α,3β-dihydroxy-20(S)-(3-ethyl-3-hydroxypentyl)-9,10-secopregna-5,7,10(19),16-tetraene from Example 11 (hereinafter referred to as "Compound 1") and 1α,3β-dihydroxy-20(S)-(5-ethyl-5-hydroxy-3-heptyny)-9,(0-secopregna-5,7,10(19),16-tetraene from Example 6 (hereinafter referred to as "Compound 2").

(A) Experimental method

Nine-month-old W-I female rats (Imamichi Institute for Animal Reproduction) were ovariectomized to remove both ovaries. From the following day, Compound 1 or Compound 2 was orally administered every day for 8 weeks. Compound 1 was given in an amount of 0.5 μg/kg, 1 μg/kg or 2 μg/kg per day in two divided doses, while Compound 2 was given in an amount of 0.125 μg/kg, 0.25 μg/kg or 0.5 μg/kg per day in two divided doses. Twenty-four hours after the last administration, blood was drawn from each rat under ether anesthesia. The rats were then euthanized and their femurs were isolated.

The femur bone mineral density was measured using a dual X-ray bone mineral densitometer (DCS-600EX, ALOKA). The blood calcium (Ca) level was measured with a HITACHI automatic analyzer (Model 7170, Hitachi). For comparison purposes, both groups of sham operation (sham) and ovariectomy (OVX; in which rats were ovariectomized only, not followed by administration of test compounds) were similarly assayed for their blood calcium (Ca) level and bone mineral density by isolating their femurs.

Statistical processing was carried out using a software package of Statistic Analysis System (SAS). A comparison between sham operation (sham) and ovariectomy (OVX) groups was made by an unpaired t-test, while a comparison between OVX and drug treatment groups was made by Dunnett's multiple test. A P-value less than 5% was defined as a significant difference.

(B) Experimental results

The results obtained are shown in Figures 1(a) and 1(b), which demonstrate blood Ca level and femur bone

mineral density, respectively. All values are expressed as mean ± S.E. In the figures, * denotes a significant difference from the OVX group at $p<0.05$ and N.S. denotes no significant difference.

The femur bone mineral density significantly decreased in the OVX group as compared to the sham group. By treatment with Compound 1 at 1 μg/kg and 2 μg/kg, the OVX-induced decrease in bone mineral density was significantly suppressed and the level of bone mineral density remained as high as in the sham group. Likewise, by treatment with Compound 2 at 0.125 μg/kg and 0.5 μg/kg, the OVX-induced decrease in bone mineral density was significantly suppressed and the level of bone mineral density remained as high as in the sham group.

In contrast, blood calcium-increasing effect was not observed for Compounds 1 and 2 in amounts where they were found to suppress the decrease in bone mineral density.

(Reference Example) Effects of 1α,25-dihydroxyvitamin $D_3$ on bone mass reduction in osteoporosis rat model

(A) Experimental method

Seven- to nine-week-old W-I female rats (Imamichi Institute for Animal Reproduction) were ovariectomized to remove both ovaries. From the following day, 1α,25-dihydroxyvitamin $D_3$ was subcutaneously administered 5 times a week for 6 weeks in an amount of 0.01 to 0.04 μg/kg per dose. After the last administration, 24-hour urine was sampled from each rat and blood was drawn under ether anesthesia. The rats were then euthanized and their lumbar vertebrae were isolated.

The lumbar bone mineral density was measured at the third lumbar vertebra site using a dual X-ray bone mineral densitometer (DCS-600, ALOKA). The blood and urinary calcium levels and the urinary creatinine level were measured with a HITACHI automatic analyzer (Model 7170, Hitachi).

Statistical processing was carried out using a software package of Statistic Analysis System (SAS). A comparison between sham operation (sham) and ovariectomy (OVX) groups was made by an unpaired t-test, while a comparison between OVX and drug treatment groups or between sham and drug treatment groups was made by Dunnett's multiple test. A P-value less than 5% was defined as a significant difference.

(B) Experimental results

The results obtained are shown in Figures 2(a) to 2(c), which demonstrate urinary Ca excretion, blood Ca level and bone mineral density, respectively, in treatment experiments with 1α,25-dihydroxyvitamin $D_3$ ($1,25(OH)_2D_3$).

The bone mineral density of each rat was calculated as percentage (%) relative to the mean bone mineral density for the sham group (=100%). All values are expressed as mean ± S.E. In the figures, * denotes a significant difference from the OVX group at $p<0.05$ and # denotes a significant difference from the sham group at $p<0.05$. N.S. denotes no significant difference.

In the OVX group, the lumbar bone mineral density decreased by about 12%. By treatment with 1α,25-dihydroxyvitamin $D_3$ at 0.04 μg/kg, on the other hand, the OVX-induced decrease in bone mineral density was significantly suppressed. However, 1α,25-dihydroxyvitamin $D_3$ at 0.04 μg/kg significantly increased both the blood Ca level and the urinary Ca excretion, as compared to the sham and OVX groups. Thus, 1α,25-dihydroxyvitamin $D_3$ was found to have an effect on bone mineral density simultaneously with blood and urinary calcium-increasing effects. Similar results were observed in oral administration (0.1 μg/kg).

INDUSTRIAL APPLICABILITY

The compounds of the present invention are useful as therapeutic agents for osteoporosis because they have an excellent bone mass-increasing effect, but almost no blood calcium-increasing effect.

## Claims

**1.** A therapeutic agent for osteoporosis which comprises, as an active ingredient, a compound of Formula (1):

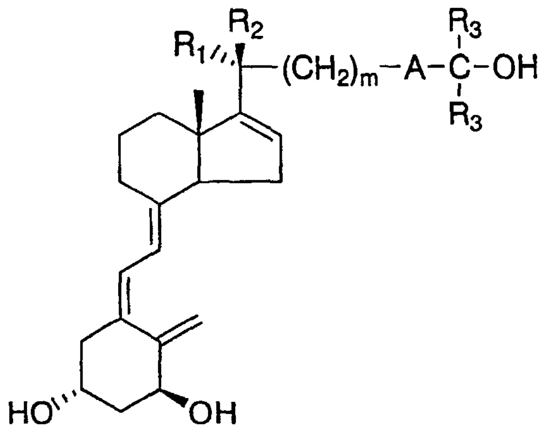

Formula (1)

wherein

$R_1$ and $R_2$ each represent a hydrogen atom or a methyl group,
$R_3$ represents a $C_1$-$C_4$ alkyl group which may be substituted with a fluorine atom,
m represents an integer of 0 to 3, and
A represents $-CH_2-CH_2-$, -CH=CH- or -C≡C-.

**2.** The therapeutic agent for osteoporosis according to claim 1, which comprises, as an active ingredient, a compound of Formula (1) wherein $R_1$ is a hydrogen atom and $R_2$ is a methyl group.

**3.** The therapeutic agent for osteoporosis according to claim 1, which comprises, as an active ingredient, a compound of Formula (1) wherein $R_1$ is a hydrogen atom, $R_2$ is a methyl group, $R_3$ represents a $C_1$-$C_3$ alkyl group which may be substituted with a fluorine atom, m represents an integer of 0 to 3, and A is -CH=CH- or -C≡C-.

**4.** The therapeutic agent for osteoporosis according to claim 1. which comprises, as an active ingredient, a compound of Formula (1) wherein $R_1$ is a hydrogen atom, $R_2$ is a methyl group, $R_3$ represents a $C_1$-$C_3$ alkyl group, m represents an integer of 2 or 3, and A is -CH=CH- or -C≡C-.

**5.** The therapeutic agent for osteoporosis according to claim 1, wherein the compound of Formula (1) is 1α,3β-dihydroxy-20(S)-(5-ethyl-5-hydroxy-3-heptynyl)-9,10-secopregna-5,7,10(19),16-tetraene.

**6.** The therapeutic agent for osteoporosis according to claim 1, which comprises, as an active ingredient, a compound of Formula (1) wherein $R_1$ is a hydrogen atom, $R_2$ is a methyl group, $R_3$ represents a $C_1$-$C_3$ alkyl group which may be substituted with a fluorine atom, m represents an integer of 0 to 3, and A is $-CH_2-CH_2-$.

**7.** The therapeutic agent for osteoporosis according to claim 1, which comprises, as an active ingredient, a compound of Formula (1) wherein $R_1$ is a hydrogen atom, $R_2$ is a methyl group, $R_3$ represents a $C_1$-$C_3$ alkyl group, m represents 0, and A is $-CH_2-CH_2-$.

**8.** The therapeutic agent for osteoporosis according to claim 1, wherein the compound of Formula (1) is 1α,3β-dihydroxy-20(S)-(3-ethyl-3-hydroxypentyl)-9,10-secopregna-5,7,10(19),16-tetraene.

**9.** A vitamin D derivative of the following Formula (2):

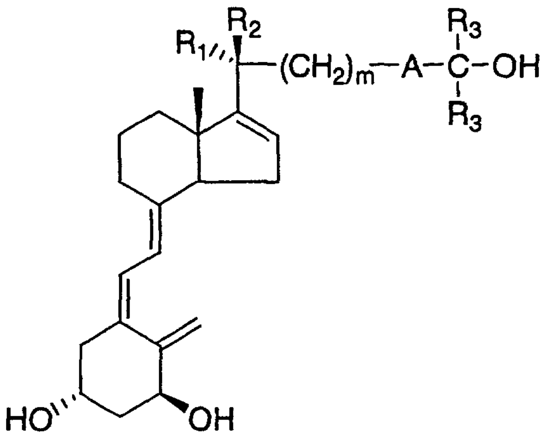

Formula (2)

wherein

$R_1$ and $R_2$ each represent a hydrogen atom or a methyl group,
$R_3$ represents a $C_1$-$C_4$ alkyl group which may be substituted with a fluorine atom,
m represents an integer of 2 or 3, and
A represents -CH=CH- or -C≡C-.

**10.** The vitamin D derivative according to claim 9, wherein in Formula (2), $R_1$ is a hydrogen atom and $R_2$ is a methyl group.

**11.** The vitamin D derivative according to claim 9, wherein the compound of Formula (2) is 1α,3β-dihydroxy-20(S)-(5-ethyl-5-hydroxy-3-heptynyl)-9,10-secopregna-5,7,10(19),16-tetraene.

**12.** A pharmaceutical composition comprising the vitamin D derivative according to any one of claims 9 to 11 as an active ingredient.

**13.** A method for treating osteoporosis, which comprises the step of administering to a patient in need of such treatment a therapeutically effective amount of a compound of Formula (1):

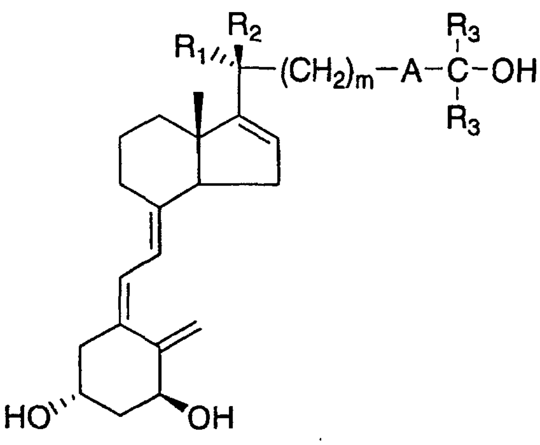

Formula (1)

wherein

$R_1$ and $R_2$ each represent a hydrogen atom or a methyl group,
$R_3$ represents a $C_1$-$C_4$ alkyl group which may be substituted with a fluorine atom,
m represents an integer of 0 to 3, and
A represents $-CH_2-CH_2-$, -CH=CH- or $-C\equiv C-$.

# Fig. 1

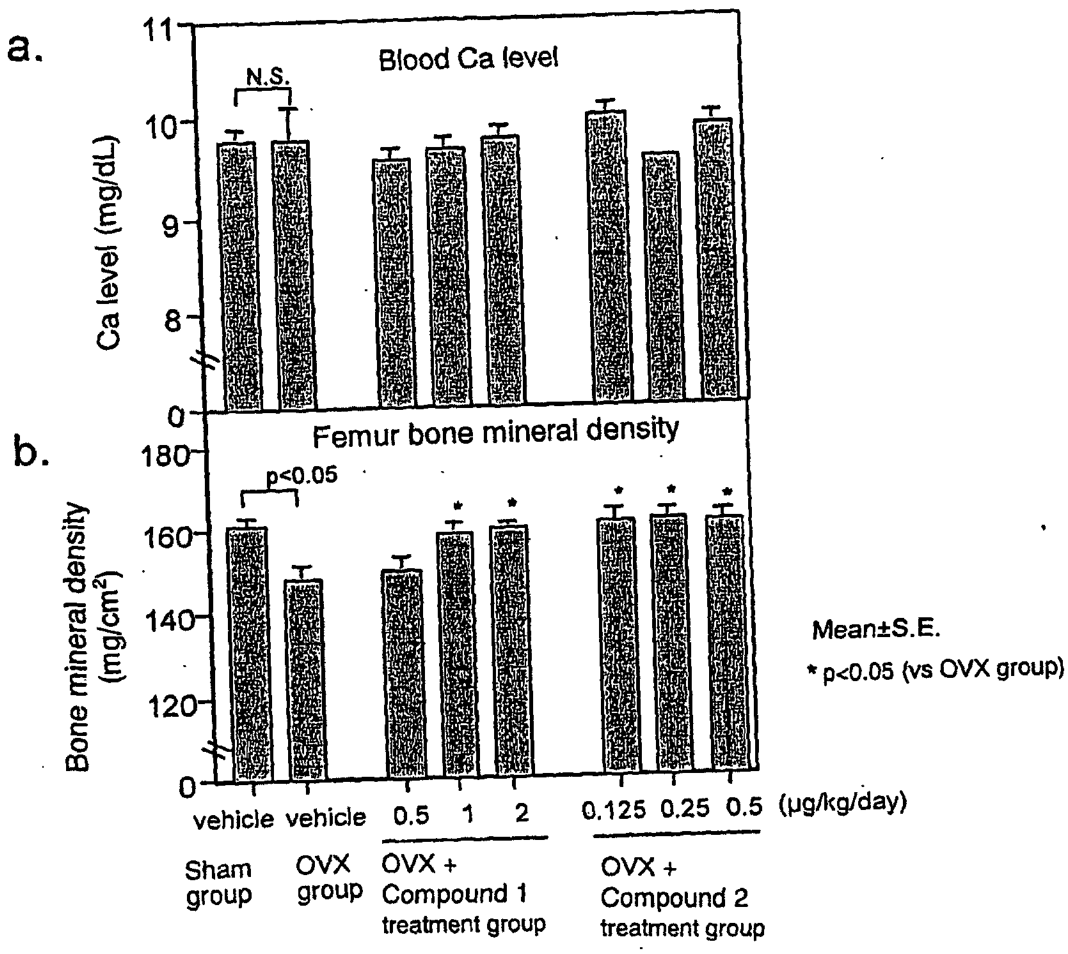
a.
Blood Ca level
N.S.
Ca level (mg/dL)
11
10
9
8
0
b.
Femur bone mineral density
p<0.05
Bone mineral density (mg/cm2)
180
160
140
120
0
vehicle vehicle 0.5 1 2 0.125 0.25 0.5 (μg/kg/day)
Sham group
OVX group
OVX + Compound 1 treatment group
OVX + Compound 2 treatment group
Mean±S.E.
* p<0.05 (vs OVX group)

# Fig. 2

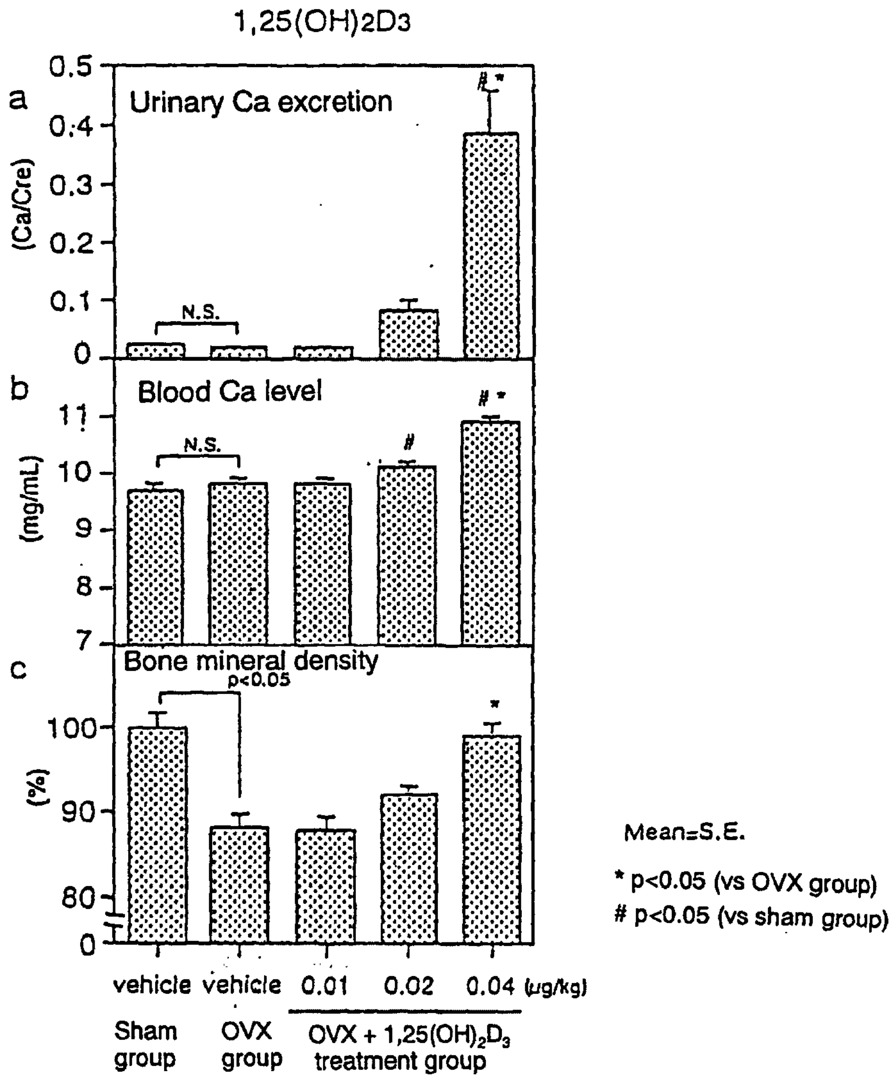
1,25(OH)2D3
a
Urinary Ca excretion
(Ca/Cre)
0.5
0.4
0.3
0.2
0.1
0
N.S.
#*
b
Blood Ca level
(mg/mL)
11
10
9
8
7
N.S.
#
#*
c
Bone mineral density
p<0.05
(%)
100
90
80
0
*
vehicle
vehicle
0.01
0.02
0.04 (µg/kg)
Sham group
OVX group
OVX + 1,25(OH)2D3 treatment group
Mean±S.E.
* p<0.05 (vs OVX group)
# p<0.05 (vs sham group)

**INTERNATIONAL SEARCH REPORT**

International application No.
PCT/JP01/07051

A. CLASSIFICATION OF SUBJECT MATTER
Int.Cl[7] A61K31/593, A61P19/10, 3/02

According to International Patent Classification (IPC) or to both national classification and IPC

B. FIELDS SEARCHED

Minimum documentation searched (classification system followed by classification symbols)
Int.Cl[7] A61K31/59, A61P19/10, 3/02

Documentation searched other than minimum documentation to the extent that such documents are included in the fields searched

Electronic data base consulted during the international search (name of data base and, where practicable, search terms used)
CAPLUS (STN), REGISTRY (STN), MEDLINE (STN), EMBASE (STN)

C. DOCUMENTS CONSIDERED TO BE RELEVANT

| Category* | Citation of document, with indication, where appropriate, of the relevant passages | Relevant to claim No. |
|---|---|---|
| X<br>Y | EP 808832 A2 (Hoffmann La Roche),<br>26 November, 1997 (26.11.97),<br>& JP 10-45714 A | 1-8,9-12<br>1-8,9-12 |
| Y | WO 93/12081 A1 (Schering Aktiengesellschaft),<br>24 June, 1993 (24.06.93),<br>& EP 637299 A1 & US 5446035 A<br>& JP 7-505132 A | 1-8,9-12 |
| Y | WO 94/00428 A1 (Schering Aktiengesellschaft),<br>06 January, 1994 (06.01.94),<br>& EP 647219 A1 & US 5585368 A<br>& US 5700791 A & JP 8-501531 A | 1-8,9-12 |
| Y | WO 98/51663 A2 (F. Hoffmann-La Roche AG),<br>19 November, 1998 (19.11.98),<br>& US 2001007907 A | 1-8,9-12 |
| P,X | WO 01/04089 A1 (F. Hoffmann-La Roche AG),<br>18 January, 2001 (18.01.01) (Family: none) | 1-8,9-12 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 November, 2001 (02.11.01) | 13 November, 2001 (13.11.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/07051

Continuation of Box No. II of continuation of first sheet (1)

The subject matter of claims 1 to 8 relates to "an osteoporosis remedy" characterized by containing as the active ingredient a compound represented by the general formula (1) shown therein.

In contrast, the subject matters of claims 9 to 12 pertain to compounds themselves which have a chemical structure within the scope of the compounds represented by the general formula (1). However, these compounds are limited to that part of the compounds (1) which is represented by the general formula (2). The subject matters of claims 9 to 12 further relate to an unspecified medicinal composition containing any of these compounds as the active ingredient, and do not necessarily relate only to an application as "an osteoporosis remedy."

It is hence considered that this international application involves two inventive concepts, i.e., an invention relating to a medicinal application as an osteoporosis remedy and an invention relating to specific novel compounds and an application thereof. These two inventions are not considered to be a group of inventions so linked as to form a single general inventive concept.

Form PCT/ISA/210 (extra sheet) (July 1992)

## INTERNATIONAL SEARCH REPORT

International application No.
PCT/JP01/07051

**Box I Observations where certain claims were found unsearchable (Continuation of item 1 of first sheet)**

This international search report has not been established in respect of certain claims under Article 17(2)(a) for the following reasons:

1. ☒ Claims Nos.: 13
because they relate to subject matter not required to be searched by this Authority, namely:

Claim 13 falls under the category of methods for treatment and diagnosis of the human body by therapy and thus relates to a subject matter which this International Searching Authority is not required, under the provisions of Article 17(2)(a)(i) of the PCT and Rule 39(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

(See extra sheet.)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☒ No protest accompanied the payment of additional search fees.

Form PCT/ISA/210 (continuation of first sheet (1)) (July 1992)